(19) Europäisches Patentamt
European Patent Office
Office européen des brevets

(11) EP 1 966 168 B1

(12) **FASCICULE DE BREVET EUROPEEN**

(45) Date de publication et mention de la délivrance du brevet:
**01.01.2014 Bulletin 2014/01**

(21) Numéro de dépôt: **06819809.2**

(22) Date de dépôt: **28.11.2006**

(51) Int Cl.:
***C07D 251/34*** (2006.01) ***C08G 18/38*** (2006.01)

(86) Numéro de dépôt international:
**PCT/EP2006/068987**

(87) Numéro de publication internationale:
**WO 2007/060251 (31.05.2007 Gazette 2007/22)**

(54) **COMPOSE A FONCTIONNALITE ISOCYANATE, SA PREPARATION ET SON UTILISATION DANS UN PROCEDE DE PREPARATION D'UN REVETEMENT**

VERBINDUNG MIT ISOCYANATFUNKTIONALITÄT, DESSEN HERSTELLUNG UND DESSEN VERWENDUNG BEI EINEM VERFAHREN ZUR HERSTELLUNG EINER BESCHICHTUNG

COMPOUND HAVING ISOCYANATE FUNCTIONALITY, PREPARATION THEREOF AND USE THEREOF IN A PROCESS FOR PREPARING A COATING

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **28.11.2005 FR 0512036**

(43) Date de publication de la demande:
**10.09.2008 Bulletin 2008/37**

(73) Titulaire: **Vencorex France**
**69800 Saint-Priest (FR)**

(72) Inventeurs:
- **BERNARD, Jean-Marie**
**F-69440 Saint-Laurent D'Agny (FR)**
- **BARBEAU, Philippe**
**F-69740 Genas (FR)**
- **OLIER, Philippe**
**F-69007 Lyon (FR)**

(74) Mandataire: **Colombet, Alain André et al**
**Cabinet Lavoix**
**62, rue de Bonnel**
**69448 Lyon Cedex 03 (FR)**

(56) Documents cités:

| | |
|---|---|
| **WO-A-99/07765** | **WO-A-03/062296** |
| **FR-A- 1 365 331** | **GB-A- 1 130 936** |
| **US-A- 3 692 707** | **US-A- 4 698 424** |

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un composé à fonctionnalité isocyanate, sa préparation et son utilisation dans un procédé de préparation d'un revêtement, notamment de type peinture ou vernis et pour des substrats métalliques en particulier.

Les domaines de l'industrie automobile et aéronautique demandent de plus en plus des compositions pour revêtement de très haute technicité dont les conditions d'utilisation soient améliorées et qui conduisent à des produits finis de qualité.

Comme compositions de ce type on connaît des formulations de polyuréthannes à deux composants dont l'un est un durcisseur du type polyisocyanate et l'autre la résine du type polyol et qui comprend en outre les additifs nécessaires pour le revêtement. Ces composants sont formulés dans deux pots séparés pour le stockage et ils sont mélangés au moment de la fabrication du revêtement de manière à effectuer une réaction de réticulation entre le polyisocyanate et le polyol.

Pour augmenter la productivité et en particulier pour accélérer la rotation des véhicules à peindre dans les cabines de mise en peinture, on demande des formulations de peinture qui sèchent de plus en plus rapidement. Le séchage implique en fait deux phénomènes. Il y a tout d'abord un phénomène chimique qui est la formation d'un réseau polyuréthanne et qui est essentiellement la conséquence de la création des liaisons covalentes uréthannes par réaction chimique entre le durcisseur et le polyol. Il y a par ailleurs un phénomène physique qui se traduit par la vitesse à laquelle le revêtement durcit. Ce séchage, au sens physique, est lié à la nature propre des constituants utilisés et notamment à la température de transition vitreuse (Tg) de ceux-ci.

Une solution préconisée et connue pour augmenter la vitesse de séchage est d'ajouter à un durcisseur polyisocyanate aliphatique, généralement à base d'hexaméthylène di-isocyanate (HDI), une certaine quantité de polyisocyanates cycloaliphatiques notamment à base d'isophorone di-isocyanate (IPDI), comme l'isophorone di-isocyanate trimère (IPDT). Toutefois, ces durcisseurs polyisocyanates à base d'IPDI contiennent des fonctions isocyanates nettement moins réactives que celles des polyisocyanates à structures aliphatiques à base d'HDI. Si le séchage dit physique est accéléré, le processus chimique de réticulation est, par contre, nettement ralenti.

La présence de fonctions isocyanates n'ayant pas réagi dans les revêtements ainsi obtenus entraîne une plastification de ceux-ci lors des opérations de polissage et de finissage. Il y a dégradation des revêtements, voire arrachement du film de peinture, créant ainsi des défauts d'aspect ce qui nécessite une reprise.

Par ailleurs, une exposition trop rapide de ces revêtements à des conditions humides entraîne l'apparition de taches blanchâtres dues à la réaction avec l'eau des fonctions isocyanates n'ayant pas réagi, ce qui rend nécessaire une reprise là encore.

Le problème qui se pose donc est d'accélérer le séchage tout en conservant les propriétés du revêtement.

Il est connu notamment de US6730405 des polyisocyanates obtenus par polycondensation de diisocyanate ou triisocyanate et son utilisation pour améliorer les vitesses de séchage.

L'objet de l'invention est de fournir un composé qui permet d'augmenter la vitesse du séchage tout en conservant une bonne réactivité des fonctions isocyanates et donc un composé permettant de répondre au problème évoqué précédemment.

Dans ce but, l'invention concerne un composé à fonctionnalité isocyanate, présentant une fonctionnalité moyenne supérieure à 2, résultant de la réaction d'un (poly)isocyanate de fonctionnalité moyenne supérieure à 2 avec au moins un composé X comprenant :

- soit au moins une fonction $B(H)_n$ dans laquelle :

  n est un nombre égal à 1 ou 2,
  H est un hydrogène labile et
  B désigne O, S, N, N étant un azote primaire ou secondaire, -C(=O)-O, -C(=O)-N, ou encore les groupes

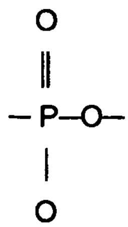

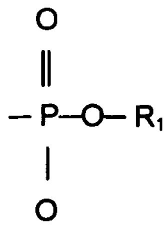

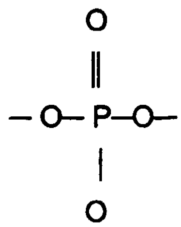

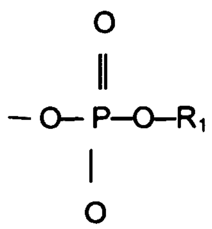

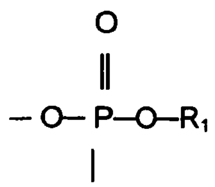

$R_1$ désignant un radical alkyle ou aralkyle, éventuellement ramifiée, ou une chaîne alkyle interrompue par un hétéroatome;

- soit au moins une fonction $B'(H)_n$ dans laquelle :

  n' est un nombre égal à 1, 2 ou 3,
  H est un hydrogène labile et B' désigne $-SiR_2R_3R_4$, $R_2$, $R_3$, $R_4$ représentant l'oxygène, un radical alkyle portant une fonction réactive avec le (poly)isocyanate, un radical aralkyle, aryle, -O-alkyle ou -O-aralkyle, le nombre de radicaux $R_2$, $R_3$, $R_4$ étant tel que n' puisse bien vérifier la condition ci-dessus;
  X étant en outre un composé cycloaliphatique, aromatique ou hétérocyclique comprenant au moins deux cycles; au moins une fonction B(H)n ou B'(H)n', de préférence toutes, sont liées directement à un carbone du cycle du composé X

et avec comme condition:

- que la réaction précitée soit conduite avec un rapport pondéral composé X/[composé X + (poly)isocyanate] d'au plus 50%.

L'invention concerne aussi une composition du type durcisseur qui est caractérisée en ce qu'elle comprend au moins un composé du type ci-dessus.
D'autres caractéristiques, détails et avantages de l'invention apparaîtront encore plus complètement à la lecture de la description qui va suivre, ainsi que des divers exemples concrets mais non limitatifs destinés à l'illustrer.
On précise pour la suite de la description que, sauf indication contraire, dans les fourchettes de valeurs qui sont données, les valeurs aux bornes sont incluses.
Le composé de l'invention est à fonctionnalité isocyanate, c'est-à-dire qu'il présente dans sa structure des fonctions isocyanates, et il peut posséder en outre des fonctions de type uréthane, allophanate, urée, biuret, ester, carbonates.
En outre, ce composé présente une fonctionnalité moyenne supérieure à 2, plus particulièrement d'au moins 2,5 et encore plus particulièrement d'au moins 2,8. Selon des modes de réalisation préférés, cette fonctionnalité moyenne peut être d'au moins 3, plus particulièrement d'au moins 3,2 et, d'une manière encore plus préférentielle, d'au moins 3,5. Cette fonctionnalité moyenne est généralement d'au plus 20, plus particulièrement d'au plus 15 et encore plus particulièrement d'au plus 10.
Pour ce composé on entend ici et pour le reste de la description par fonctionnalité moyenne et pour la fonction isocyanate le nombre r :

$$r = Mn \times [\ I\ ]$$

dans lequel Mn est la masse molaire moyenne en nombre du composé à fonctionnalité isocyanate, [ I ] est la concentration en fonction isocyanate exprimée en mole/g.
La masse Mn est déterminée par chromatographie par perméation de gel.
Le composé de l'invention est le produit de la réaction d'au moins un composé X précité avec un (poly)isocyanate. On doit donc comprendre que l'invention s'applique au cas où l'on fait réagir le (poly)isocyanate avec un mélange de plusieurs composés X.
Comme indiqué plus haut, le composé X doit présenter plusieurs caractéristiques.
Il doit tout d'abord comprendre au moins une fonction B(H)n ou B'(H)n' dans laquelle H est un hydrogène labile et B désigne différents atomes ou groupe d'atomes.
Ainsi, B peut désigner O, la fonction BH étant OH dans ce cas; S, cas correspondant à la fonction SH; N, l'azote

étant primaire ou secondaire, c'est à dire que l'atome d'azote porte deux ou un atome d'hydrogène respectivement et ce qui correspond aux fonctions $NH_2$ ou NH respectivement, B pouvant être encore le groupement C(=O)-N, ce qui correspond pour BH à la fonction C(=O)-NH ou C(=O)-$NH_2$ ou encore C(=O)-NHR', R' étant une chaîne alkyle, éventuellement ramifiée, ou aryle, de 1 à 10 atomes de carbone généralement. Les valeurs de B qui ont été mentionnées ici correspondent à des modes de réalisation préférentiels de l'invention.

Par ailleurs, comme indiqué plus haut, B peut désigner un groupe comprenant du phosphore, auquel cas, le composé X est par exemple un composé de formule

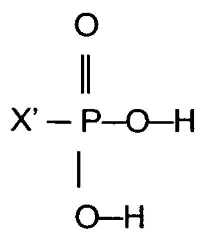

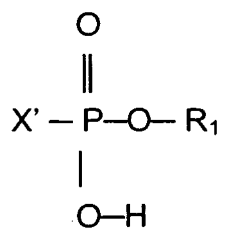

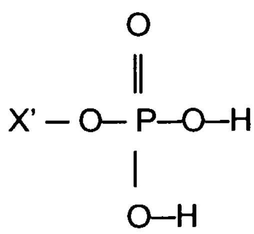

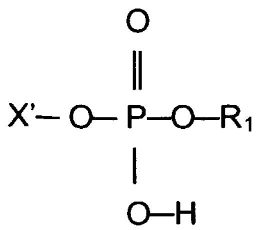

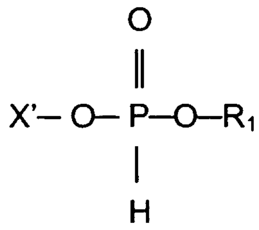

dans laquelle X' est le reste du composé X et R1 est tel que défini plus haut, en précisant ici que le radical alkyle ou aralkyle comprend plus particulièrement au plus 20 atomes de carbone, notamment de 1 à 10 atomes de carbone. Dans le cas où $R_1$ désigne une chaîne alkyle interrompue par un hétéroatome, cette chaîne comporte un nombre de carbone qui est généralement d'au plus 60, plus particulièrement d'au plus 40 et encore plus particulièrement d'au plus 35. Dans ce même cas, l'hétéroatome peut être plus particulièrement l'oxygène et le nombre d'hétéroatomes est de préférence compris entre 1 et 20. Le groupe B tel que défini dans le présent paragraphe est avantageux dans le cas de l'utilisation des composés de l'invention dans des compositions en phase aqueuse.

On doit noter enfin que l'invention s'applique bien entendu aux cas où X comprend plusieurs fonctions B(H)n ou B'(H)n', ces fonctions pouvant être identiques ou différentes.

Ce composé X présente plus particulièrement une fonctionnalité moyenne égale ou supérieure à 1 qui peut être par exemple comprise entre 1 et 10 et plus particulièrement entre 1 et 5 et avantageusement entre 1 et 3.

Cette fonctionnalité moyenne du composé X est donnée par le nombre r' :

$$r' = M'n \times [B(H)_n] \text{ ou } r' = M'n \times [B'(H)_{n'}]$$

dans lequel M'n est la masse molaire moyenne en nombre du composé X, $[B(H)_n]$ et $[B'(H)_{n'}]$ représentent les concentrations respectives en fonction $B(H)_n$ et $B'(H)_n$ exprimées en mole/g. La masse M'n est déterminée par chromatographie par perméation de gel. La concentration en fonction $B(H)_n$ ou $B'(H)_n$ est calculée par une méthode de potentiométrie directe dans le cas des fonctions pour lesquelles B est N, -C(=O)-O, -C(=O)-N et les groupes contenant du phosphore précités ou indirecte pour les autres fonctions, cette méthode indirecte consistant à faire réagir la fonction $B(H)_n$ ou $B'(H)_{n'}$ avec de l'anhydride acétique puis à doser en retour l'acide acétique libéré. Une méthode d'analyse, comme la RMN, peut aussi être utilisée pour déterminer cette concentration.

Le composé X est par ailleurs un composé organique à structure cycloaliphatique, aromatique ou hétérocyclique. Les cycles constituant la structure du composé X peuvent être fusionnés ou liés entre eux par des chaînes aliphatiques ou encore par une simple liaison. Ceci est le cas, comme indiqué plus haut lorsque B désigne un azote secondaire. Dans le cas de chaînes aliphatiques, celles-ci sont de préférence courtes et éventuellement ramifiées, par exemple des chaînes d'au plus 15 atomes de carbone, notamment d'au plus 10, plus particulièrement d'au plus 6 et encore plus particulièrement d'au plus 4 atomes de carbone. Ces cycles peuvent comporter des chaînes alkyles courtes, éventuellement ramifiées, par exemple des chaînes d'au plus 10 atomes de carbone, notamment d'au plus 6 atomes, plus particulièrement d'au plus 4 atomes et encore plus particulièrement d'au plus 2 atomes. Les cycles peuvent être aussi

des bicycles.

Le composé X comporte au moins deux cycles, plus particulièrement au moins trois. Dans le cas de la mise en oeuvre d'un mélange de composés X, il est préférable qu'au moins un de ces composés soit un composé à au moins deux cycles.

Il peut s'agir enfin d'un composé sous forme de particules à base d'un polymère réticulé.

Pour le choix du composé X on retiendra préférentiellement les composés à structure rigide. On entend par là les structures qui sont stériquement encombrées ou les structures à mobilité conformationnelle réduite ou qui sont susceptibles de développer des liaisons inter ou intra moléculaires fortes pouvant même conduire à des taux de cristallinité élevés, ainsi que des structures réticulées, ou enfin des composés à des températures de fusion ou à Tg élevées, par exemple une Tg d'au moins 0°C, plus particulièrement d'au moins 20°C et encore plus particulièrement d'au moins 40°C.

De manière à avoir une structure rigide au sens donné ci-dessus, on pourra utiliser tout particulièrement les composés X dans lesquels au moins une et de préférence toutes les fonctions $B(H)_n$ ou $B'(H)_{n'}$ sont liées directement à un carbone du cycle du composé X. Ces produits, après réaction avec le (poly)isocyanate, donnent des structures à mobilité conformationnelle réduite. Selon un mode de réalisation particulier lorsque X ne présente qu'un seul cycle, la fonction $B(H)_n$ ou $B'(H)_n$ est liée directement à un carbone de ce cycle.

Selon un mode de réalisation préféré de l'invention, la fonction $B(H)_n$ est la fonction OH. Dans ce cas, on préfère les composés à fonction OH secondaire ou à fonction OH primaire mais encombrée. On entend par « encombrée » une fonction dont le carbone en bêta de la fonction OH est ramifié et porteur au moins d'un groupe alkyle avec au moins un atome de carbone. Comme exemple d'un tel cas on peut citer les structures néopentylique ou isobutylique.

Le composé X peut ainsi être choisi parmi les diols.

On va donner ci-dessous des exemples de composés du type diol qui conviennent bien dans la mise en oeuvre de l'invention.

Ainsi, le composé X peut être choisi parmi les bisphénols, éventuellement substitués, notamment A et F, et leurs dérivés hydrogénés. On peut citer notamment le bisphénol A hydrogéné.

On peut aussi mentionner les dérivés polyphénoliques à pont éther des bisphénols. On entend par là les produits de formule (1) ou (1'):

$$BP\text{-}[\text{-}(O\text{-}CH_2\text{-}CH_2\text{-})_n\text{-}O\text{-}\Phi\text{-}R_5\text{-}\Phi\text{-}]_m\text{-}OH \qquad (1)$$

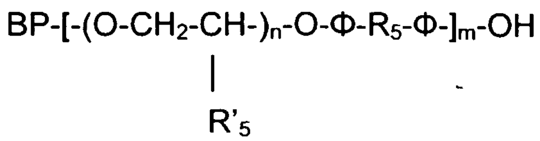
(1')

dans laquelle BP désigne le reste d'un radical bisphéno), Φ un noyau benzénique, $R_5$ un radical alkyle linéaire ou ramifié, $R'_5$ un radical alkyle en C1-C5, tel que le méthyle, et n et m des nombres entiers, étant entendu que n doit de préférence avoir une valeur faible pour conserver au produit une structure rigide, par exemple d'au plus 5, de préférence d'au plus 3, et m pouvant être par exemple compris entre 1 et 10.

Les dérivés hydrogénés de ces produits polyphénoliques peuvent aussi être utilisés.

On peut aussi mentionner comme composés X à fonction OH, les dérivés du cyclopentadiène, du dicyclopentadiène et du tricyclopentadiène, qui peuvent être obtenus par une réaction d'hydroformylation de ceux-ci, du dicyclopentadiène par exemple, suivi d'une hydrogénation pour obtenir l'alcool polycyclique correspondant, ou encore les dérivés des terpènes comme le terpénylcyclohexanol, et les dérivés de la série des terpènes comme les isobornyl, isocamphyl et isofenchyl. On peut citer comme exemple de ces dérivés le norbornadiène, le 5-éthylidène-2-norbornène, le limonène.

On peut utiliser les cyclanes à fonction OH, plus particulièrement le cyclohexane-diol, le tricyclodécane-diméthanol, le tricyclodécane-diol, le tricyclohexylméthanol ou encore les dérivés du terpénylcyclohexanol ou de l'isobornylcyclohexanol, les dérivés de l'adamantane pour les tricycles condensés ou la décaline-diol dans les bicycles fusionnés.

En outre, conviennent aussi dans le cadre de l'invention, comme composés X, les produits de réaction d'acides carboxyliques avec des composés à fonction hydroxyle masquée. Par composés à fonction hydroxyle masquée, on entend les composés à fonctions époxy; carbonate et dans ce cas, les fonctions carbonates cycliques de préférence, comme le carbonate de glycérol ; ou encore les composés à fonction dioxolane. Pour cette dernière fonction, on peut citer comme composé le 2,2 diméthyl 1,3 dioxolane 4 méthanol. La réaction concernée ici peut être soit une estérification entre les fonctions hydroxyles masquées et les fonctions carboxyliques, soit une réaction par ouverture de la fonction hydroxyle masquée avec libération de la fonction hydroxyle. Pour ce dernier cas, on peut citer plus particulièrement la réaction des acides carboxyliques avec les composés à fonction époxy.

Les acides carboxyliques peuvent être notamment des acides de formule $R_4COOH$ dans laquelle $R_4$ est un radical aliphatique, cyclique, polycyclique, aromatique ou hétérocyclique, éventuellement ramifié ou substitué.

Les composés à fonction époxy peuvent être aliphatiques, cycloaliphatiques ou hétérocycliques et peuvent contenir au moins une fonction dérivée des fonctions carboxyliques (fonctions ester ou amide). Ces composés peuvent porter éventuellement des substituants qui sont des chaînes alkyles éventuellement ramifiées. On préfère les composés époxy possédant au moins un cycle.

A titre d'exemples, on peut citer pour les acides carboxyliques les acides acétique, propionique, isobutyrique, triméthyl 2,2,2 acétique (pivalique), benzoïque, cyclohexanoïque, téréphtalique, phtalique, cyclohexanedicarboxylique.

Toujours à titre d'exemples, on peut citer pour les composés époxy l'oxyde de styrène, l'epychlorhydrine et ses dérivés, l'oxyde de cyclohexène, l'exo-2,3 époxynorbomane (bicycle), le 3,4 époxycyclohexanecarboxylate de l'hydroxyméthyl 3,4 époxycyclohexane, le méthyl 3,4 époxycyclohexane carboxylate, le bis 3,4 époxycyclohexanecarboxylate de 1,3 diméthylpropane 1,3 diol.

Comme composé X à fonction OH, on peut aussi mettre en oeuvre le produit de la réaction d'un composé à fonction(s) époxy avec un phosphate de formule (2) $(O)P(OR_6)(OR_7)(OR_8)$ dans laquelle $R_6$, $R_7$, $R_8$, sont identiques ou différents et désignent l'hydrogène, un radical alkyle, linéaire ou ramifié, notamment comprenant de 1 à 25 atomes de carbone, un radical cycloaliphatique, un radical aromatique, un radical aralkyle, une chaîne polyoxyalkylène dont le nombre de motifs oxyalkylènes linéaires ou ramifiés est compris entre 1 et 25 et dont le nombre de carbones de la chaîne alkylène est compris entre 2 et 6, cette chaîne polyoxyalkylène pouvant être, de préférence, substituée sur ses fonctions terminales par une chaîne alkyle, linéaire ou ramifiée, de préférence en $C_1$-$C_{20}$ ou par une chaîne aralkyle, éventuellement ramifiée, dont le nombre de carbones peut être compris entre 7 et 20; et avec comme condition dans la formule (2) qu'au moins un de $R_6$, $R_7$, $R_8$ soit un atome d'hydrogène. Pour les composés de formule (2) on préfère ceux comportant un cycle.

On peut aussi utiliser comme composé X le produit de la réaction d'un composé à fonction époxy avec un polyaminoéther de formule (3) $R_9$-[-O-$CH_2$-

$$CH_2\text{-}O\text{-}]_p\text{-}NH_2 \text{ ou (3') } R_9\text{-}[\text{-}O\text{-}CH_2\text{-}\underset{\displaystyle R'_9}{\underset{|}{CH}}\text{-}O\text{-}]_p\text{-}NH_2$$

ou encore de formule (4) $R_9$-[-O-$CH_2$-$CH_2$-O-]$_q$-$CH(CH_3)$-$CH_2$-$NH_2$, $R_9$ étant l'hydrogène, un radical alkyle, notamment en $C_1$-$C_4$, un radical $CH_2CH_2NH_2$ ou $CH_2CH(CH_3)NH_2$, $R'_9$ étant un radical alkyle, notamment en $C_1$-$C_4$, p et q étant des nombres entiers compris entre 2 et 10, de préférence entre 2 et 5; ou encore ie produit de la réaction d'un composé à fonction époxy avec la morpholine ou un dérivé de celle-ci.

Comme indiqué plus haut, le composé de l'invention résulte de la réaction d'un composé X tel que décrit ci-dessus avec un (poly)isocyanate de fonctionnalité moyenne supérieure à 2.

Par (poly)isocyanate de fonctionnalité moyenne supérieure à 2, on entend les composés qui présentent au moins un cycle isocyanurate, un motif biuret, une fonction allophanate ou encore une fonction acylurée. On entend également les composés qui présentent au moins un cycle uretidinedione, et les isomères des isocyanurates, tels que l'iminooxadiazinedione. Ces composés peuvent être obtenus par homo ou hétérocondensation de monomères choisis parmi les monomères isocyanates aliphatiques, cycloaliphatiques, arylaliphatiques, aromatiques et hétérocycliques et notamment parmi ceux-ci les di-isocyanates et les tri- isocyanates;

Comme exemples non limitatifs d'isocyanates aliphatiques et cycloaliphatiques on peut citer les produits suivants :

- 1,6-hexaméthylène di-isocyanate (HDI),
- 1,12-dodécane di-isocyanate,
- cyclobutane-1,3- di-isocyanate,
- cyclohexane-1,3 et/ou 1,4- di-isocyanate,
- 1,-isocyanato-3,3,5-triméthyl-5-isocyanatométhyl cyclohexane (isophorone di-isocyanate) (IPDI),
- les isocyanatométhyl di-isocyanates, notamment le 4-isocyanatométhyl-1-8 di-isocyanate (TTI),
- 2,4 et/ou 2,6-hexahydrotoluylène di-isocyanate ($H_6TDI$),
- hexahydro-1,3 et/ou 1,4-phénylène di-isocyanate,
- perhydro 2,4' et/ou 4,4' -diphénylméthane di isocyanate ($H_{12}MDI$),
  et en général les produits d'hydrogénation des précurseurs aromatiques, amines ou carbamates,
- les bis-isocyanométhylcyclohexane (notamment 1,3 et 1,4) (BIC),
- les bis-isocyanométhylnorbomane (NBDI),

- 2-méthylpentaméthylène 1, 5 di isocyanate (MPDI)
- les tétraméthylxylilène di isocyanate (TMXDI)
- les esters de la lysine di- ou tri isocyanate (LDI ou LTI).
- les triisocyanates tels que le 4 isocyanato méthyle - 1,8 octaméthylène diisocyanate

Comme exemples non limitatifs d'isocyanates aromatiques, on peut citer :

- le 2,4- et/ou le 2,6-toluylène di-isocyanate (TDI),
- le diphénylméthane-2,4' et/ou 4,4'-di-isocyanate (MDI),
- le 1,3- et/ou le 1,4-phénylène di-isocyanate,
- le triphénylméthane-4,4',4"-triisocyanate, et
- les oligomères du MDI ou du TDI.

On préfère cependant n'utiliser qu'en quantités limités les dérivés aromatiques car ceux-ci peuvent conduire à des revêtements qui peuvent subir une coloration au cours du vieillissement, notamment en cas d'exposition au rayonnement ultra violet, par exemple l'ultra violet solaire. C'est pourquoi, les dérivés aliphatiques et cycloaliphatiques dont au moins une des fonctions NCO, de préférence deux, n'est pas directement greffée sur le cycle aliphatique sont généralement préférés.

Comme on le verra plus loin, il est aussi possible d'utiliser des (poly)isocyanates tels que définis ci-dessus et qui sont en outre rendus hydrophiles par greffage d'un additif d'hydrophilisation convenable.

On utilise plus particulièrement dans le cadre de la présente invention les (poly)isocyanates présentant une fonctionnalité moyenne d'au moins 2,5 et encore plus préférentiellement d'au moins 3. Généralement, la fonctionnalité moyenne est d'au plus 30, plus particulièrement d'au plus 15 et encore plus particulièrement d'au plus 10, par exemple elle peut être comprise entre 3 et 6.

Les (poly)isocyanates peuvent être utilisés en mélange avec les monomères précités mais, dans ce cas, la teneur en monomère est d'au plus 50%, notamment d'au plus 30%, plus particulièrement d'au plus 20% et encore plus particulièrement d'au plus 10%.

On préfère aussi utiliser les (poly)isocyanates de faible viscosité, c'est-à-dire de viscosité d'au plus 40000 mPa.s, plus particulièrement d'au plus 20000 mPa.s, encore plus particulièrement d'au plus 10000 mPa.s, de préférence d'au plus 5000 mPa.s et encore plus préférentiellement d'au plus 2500 mPa.s. Cette viscosité correspond à une mesure faite à 100% d'extrait sec à 25°C.

Les composés à fonctionnalité isocyanate de l'invention sont obtenus en faisant réagir un composé X avec un (poly)isocyanate du type de ceux décrits ci-dessus.

Cette réaction est conduite avec un rapport pondéral composé X/[composé X + (poly)isocyanate] d'au plus 50%. Ce rapport peut être notamment d'au plus 40%, plus particulièrement d'au plus 25% et encore plus particulièrement d'au plus 20%. Généralement il est d'au moins 1%, plus particulièrement d'au moins 2% et encore plus particulièrement d'au moins 5%.

On choisit les valeurs respectives des fonctionnalités du composé X et du (poly)isocyanate d'une manière telle qu'à l'issue de la réaction on obtient un composé qui doit pouvoir être formulable, c'est-à-dire utilisable dans les conditions de l'application recherchée. On entend par formulable un produit qui généralement présente à 70% d'extrait sec et à 25°C une viscosité d'au plus 20000 mPa.s, plus particulièrement d'au plus 10000 mPa.s et de préférence d'au plus 6000 mPa.s. On doit bien comprendre que cette valeur n'est donnée ici qu'à titre d'exemple et qu'elle ne peut pas être considérée comme limitative.

Les conditions dans lesquelles peut être conduite la réaction entre le composé X et (poly)isocyanate vont maintenant être décrites plus en détail.

En général, le procédé est conduit de telle manière que le ratio molaire $B(H)_n/NCO$ soit compris entre 1 et 50 % de préférence entre 2 et 30 % et avantageusement entre 3 et 25%. Ces ratios sont choisis par l'homme du métier en fonction de la masse moléculaire, de la fonctionnalité de chacun des produits engagés et des.composés que l'on cherche à obtenir.

D'une manière générale aussi, la synthèse des composés de l'invention est réalisée selon une réaction classique des fonctions isocyanates avec les fonctions $B(H)_n$ à une température comprise entre 20 et 200°C, de préférence à une température comprise entre 25 et 150°C. Dans certains cas, un catalyseur peut être ajouté, en général ces catalyseurs sont des composés organométalliques à activité acide de Lewis. On peut ainsi citer les dérivés de l'étain comme le dibutyl dilaurate d'étain, le dibutyl diacétate d'étain, les acétylacétonates de zirconium ou d'aluminium ou les acylates (acétate, octoate..) de bismuth, cette liste de catalyseurs n'étant pas limitative.

La quantité de catalyseur utile à l'obtention des composés de l'invention peut être comprise entre 0 et 1000 ppm, avantageusement entre 0 et 500 ppm et encore plus préférentiellement entre 0 et 250 ppm par rapport à la quantité de (poly)isocyanates.

Les synthèses sont conduites en masse ou en solvant en fonction de la viscosité du composé final obtenu. Le solvant de synthèse est généralement choisi parmi les esters tels que l'acétate de n-butyle, l'acétate de tertio-butyle, les solvants aromatiques tels que le SOLVESSO 100, les cétones tels que la méthyl-isobutyle cétone.

On peut noter qu'il est possible de procéder à une distillation du composé X ainsi que du solvant de synthèse précité pour éliminer les traces résiduelles d'eau dans le milieu réactionnel.

Après les généralités données ci-dessus, la synthèse des composés va maintenant être décrite plus en détail en fonction du type de composé à fonctionnalité isocyanate que l'on cherche à préparer. Dans la description qui suit le taux de fonctions isocyanates consommé est exprimé en % et correspond à la formule suivante :

$$(T\ NCO\ départ - T\ NCO\ arrivée / T\ NCO\ départ)x100$$

Avec T NCO départ représentant la valeur du titre en fonctions isocyanates du mélange réactionnel de départ et T NCO arrivée représentant la valeur du titre en fonctions isocyanates du mélange réactionnel au temps de mesure.

La mesure du taux de fonctions isocyanates consommées est faite selon la norme NF T 52-132 qui est une méthode par dosage avec la N,N dibutylamine (DBA).

A partir de ce dosage on peut aussi estimer le taux de transformation en composé isocyanate de départ. Lorsqu'une fonction isocyanate de ce composé isocyanate a été transformée au cours de la réaction alors on consomme aussi la molécule de cet isocyanate. Ainsi pour le monomère hexaméthylène diisocyanate qui est un diisocyanate, le taux de transformation en isocyanate correspond à environ le double de la valeur trouvée pour le titre en fonctions isocyanates.

Dans le cas d'un composé selon l'invention à fonction isocyanate mais aussi à fonction uréthanne, le procédé de préparation comporte les étapes suivantes :

(a) introduction dans un réacteur équipé d'un système d'agitation et des équipements de contrôle du ou du mélange de (poly)isocyanate(s);
(b) ajout éventuel de solvant;
(c) ajout au (poly)isocyanate ou au mélange de (poly)isocyanates du composé X ou du mélange de composés X à fonction $B(H)_n$, $(B(H)_n$ étant dans le cas présent (préparation d'un composé à fonctions isocyanate/uréthanne) une fonction OH;
(d) mise du mélange réactionnel sous courant d'un gaz inerte tel qu'azote ou argon;
(e) ajout éventuel au mélange des additifs de réaction tels que des antioxydants ou agents de stabilisation du type trialkyle ou triaryle phosphites ou composés aromatiques phénoliques encombrés tels que les 2,6 di tertio butyle phénol ou des dérivés oligomériques de ces composés;
(f) réaction à une température comprise entre 80 et 130°C jusqu'à ce que le taux de transformation en fonctions isocyanates soit égal à la quantité molaire de fonctions $B(H)_n$ à faire réagir. Ainsi dans le cas présent (formation de liaisons uréthannes) la quantité de fonctions isocyanates consommées sera égale à au moins 90 % de la quantité molaire de fonctions $B(H)_n$, de préférence supérieure à 95% de cette quantité et encore plus préférentiellement égale à la quantité totale de fonctions $B(H)_n$ introduite.
(g) récupération du produit une fois que le taux de transformation est atteint.

Dans le cas d'un composé selon l'invention à fonction isocyanate(s) mais aussi à fonction allophanate(s), le procédé de préparation met en oeuvre, là encore, un composé X (ou un mélange de composés X) à fonction OH.

Le procédé comporte les étapes (a) à (e) décrites plus haut ainsi qu'une étape d'addition, après l'étape (d), d'un catalyseur acide de Lewis (dibutyl dilaurate d'étain ou acétylacétonate de zirconium) dans les quantités indiquées ci-dessus.

Le procédé comporte en outre une étape (f) de réaction qui est conduite ici à une température comprise entre 100 et 150°C jusqu'à ce que le taux de transformation en fonctions isocyanates soit égal à environ deux fois la quantité molaire de fonctions $B(H)_n$ à faire réagir. En effet, un composé à fonction allophanate est le produit de réaction d'un composé à fonction isocyanate sur un composé à fonction uréthanne, ce dernier étant lui-même le fruit de la réaction d'un composé à fonction isocyanate sur un composé à fonction hydroxyle. Pour obtenir la transformation en allophanate on consomme donc en théorie deux moles de fonction isocyanate pour une mole de fonction OH et, en pratique ici, on conduit la réaction de manière à consommer une quantité de fonctions isocyanates égale à au moins une mole et comprise entre une et deux, ceci en fonction des propriétés que l'on cherche à obtenir pour le composé X.

La synthèse de composés à fonctionnalité isocyanate comportant en outre des fonctions urées met en jeu des composés X dont $B(H)_n$ est soit une amine primaire soit une amine secondaire. Le procédé de préparation se déroule de la même manière que pour les synthèses des composés à fonctions isocyanates et uréthannes à la différence que

la température de réaction est comprise entre la température ambiante et 80°C. La réaction ne demande pas de catalyseurs spécifiques. La réaction est arrêtée lorsque le taux de transformation en fonctions isocyanates est égal à la quantité molaire de fonctions B(H)n (dans le cas présent amine primaire ou secondaire) à faire réagir. Ainsi dans le cas présent de formation de liaisons urées, la quantité de fonctions isocyanates consommées sera égale à au moins 90 % de la quantité molaire de fonctions B(H)n ou B'(H)n', de préférence à au moins 95% de cette quantité et encore plus préférentiellement égale à la quantité totale de fonctions $B(H)_n$ introduite.

La synthèse de composés polyisocyanates à fonctionnalité isocyanate comportant en outre des fonctions biurets met en jeu des composés X dont $B(H)_n$ est soit une amine primaire soit une amine secondaire. On procède de la même manière que pour les synthèses des composés à fonctions isocyanates et uréthannes à la différence que la température de réaction est comprise entre 100 et 150°C. La réaction peut être catalysée par l'ajout de composés acides tels que l'acide propionique ou des dialkyles phosphates. Un composé biuret est le produit de réaction d'un composé isocyanate sur un composé à fonction urée et ce dernier est lui-même le produit de réaction d'un composé à fonction isocyanate sur une amine. Pour obtenir la transformation en biuret, on consomme donc en théorie deux moles de fonction isocyanate pour une mole de fonction amine et, en pratique ici, on conduit la réaction de manière à consommer une quantité de fonctions isocyanates égale à au moins une mole et comprise entre une et deux, ceci en fonction des propriétés que l'on cherche à obtenir pour le composé X.

Dans le cas des composés X dont les fonctions $B(H)_n$ sont des fonctions SH, alors on utilisera le même type de procédé que celui décrit pour l'obtention des composés à fonctions isocyanates et uréthannes. La consommation des fonctions isocyanates sera la même que pour celle des fonctions uréthannes si on s'arrête au stade des composés thio-uréthannes. Elle correspondra au taux de transformation des fonctions NCO du procédé allophanates si on cherche à obtenir des composés thioallophanates.

Pour ce qui est des composés X dont les fonctions $B(H)_n$ sont des fonctions amides le processus sera le même sauf que les conditions opératoires seront une température de réaction comprise entre 100 et 150°C, et la présence éventuelle d'un catalyseur acide de Lewis. La réaction est arrêtée lorsque le taux de transformation en fonctions isocyanates est égal à la quantité molaire de fonctions B(H)n (ici amide primaire ou secondaire) que l'on souhaite faire réagir. Les fonctions amides peuvent résulter d'une réaction entre les fonctions isocyanates et les fonctions acides.

Dans le cas de composés X à fonctions $B(H)_n$ ou $B'(H)_{n'}$ différentes on adaptera les conditions opératoires au composé que l'on désire obtenir. D'une manière générale, on notera que les conditions de réactions d'un composé à fonction isocyanate avec un composé à hydrogène labile du type composé X sont bien connues de l'homme du métier et on pourra se référer à ce sujet à l'ouvrage général « Methoden der Organischen Chemie » Houben Weyl, Georg Thieme Verlag 1983.

Dans tous les cas qui viennent d'être décrits, les structures des composés sont confirmées par des techniques d'analyse connues telles que la spectroscopie infra rouge, la spectrométrie de résonance magnétique nucléaire (RMN) du proton ou du carbone.

L'invention concerne aussi une composition ou formulation du type durcisseur qui est caractérisée en ce qu'elle comprend un composé à fonctionnalité isocyanate du type ci-dessus ou un mélange de ces composés. Ce composé peut être le seul composant de la composition du type durcisseur, néanmoins, cette composition peut comprendre en outre un (poly)isocyanate ou un mélange de (poly)isocyanate au sens donné plus haut, ce qui implique que tout ce qui a été décrit précédemment au sujet des (poly)isocyanates, tant pour leur nature que pour leur viscosité notamment s'applique aussi ici. Ce (poly)isocyanate ou mélange de (poly)isocyanate supplémentaire peut être différent de celui utilisé pour la préparation du composé à fonctionnalité isocyanate de l'invention. L'ajout de ce (poly)isocyanate ou mélange de (poly)isocyanate supplémentaire permet de faire varier notamment la viscosité de la composition et/ou son titre en fonction isocyanate. De préférence, la teneur en composé à fonctionnalité isocyanate dans la composition du type durcisseur est comprise entre 100% et 5%, plus particulièrement entre 100% et 10% et encore plus particulièrement entre 100% et 25%, cette teneur étant exprimée en poids de composé à fonctionnalité isocyanate par rapport au poids total de la composition du type durcisseur.

La composition du type durcisseur de l'invention peut être utilisée pour la préparation de revêtements, de type peinture ou vernis, en phase solvant ou en phase aqueuse.

Dans le cas d'une utilisation en phase solvant, et tout particulièrement dans le cas où l'on souhaite une stabilité au stockage de quelques jours à quelques mois, il est préférable d'utiliser un solvant non réactif. Par exemple, ce peut être un solvant du type ester (acétate de butyle), cétone, éther de glycol acylé ou dialkylé, solvants aromatiques substitués comme le xylène. Il s'agit de solvants courants dans la formulation des isocyanates, à faible quantité en eau de préférence.

Dans le cas d'une utilisation en phase aqueuse, la composition peut contenir un additif permettant de mettre celle-ci en émulsion ou de la rendre dispersable ou hydrosoluble. Plusieurs modes de réalisation et variantes peuvent être alors envisagés.

Dans le cas d'un premier mode de réalisation, la composition du type durcisseur contient un additif hydrophile du type non réactif, c'est-à-dire que cet additif est présent en mélange avec la composition sans qu'il y ait eu une réaction entre cet additif et le composé à fonctionnalité isocyanate de la composition. Comme additif ce type, on peut mentionner

ceux décrits dans les documents WO 97/31960 et FR 2855768-A1 à l'enseignement desquels on pourra se reporter. Ces additifs présentent une fonction anionique et avantageusement un fragment de chaîne polyéthylène glycol d'au moins une, de préférence d'au moins 5 unités éthylènyloxyles.

On peut mentionner plus particulièrement parmi ces additifs ceux ci-dessous :

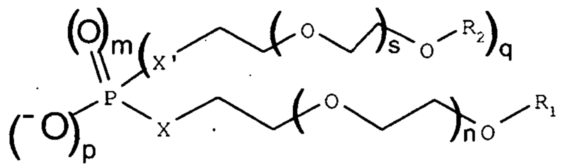

avec lorsque q est égal à zéro :

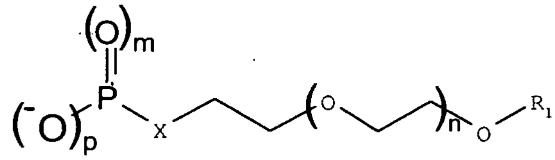

et où p représente zéro ou un entier entre 1 et 2 (intervalles fermés c'est à dire comprenant les bornes);
où m représente zéro ou un entier entre 1 et 2 (intervalles fermés c'est à dire comprenant les bornes);
où la somme p + m + q est au plus égale à trois;
où la somme 1 + p + 2m + q est égale à trois ou à cinq;
où X et X', semblables ou différents, représentent un bras comportant au plus deux chaînons carbonés;
où n et s, semblables ou différents, représentent un entier choisi entre 5 et 30 avantageusement entre 5 et 25, de préférence entre 9 et 20 (intervalles fermés c'est à dire comprenant les bornes);
où $R_1$ et $R_2$, semblables ou différents, représentent un radical hydrocarboné, avantageusement choisi parmi les aryles et les alcoyles éventuellement substitués notamment par atome d'halogène notamment fluor.

Dans le cas d'un second mode de réalisation, la composition du type durcisseur contient un additif hydrophile du type réactif, c'est-à-dire que cet additif est présent dans la composition mais en étant greffé sur le composé à fonctionnalité isocyanate de la composition. Dans le cas de ce second mode de réalisation plusieurs variantes peuvent alors être envisagées.

Selon une première variante, on choisit un composé X spécifique, c'est-à-dire un composé X hydrophile que l'on fait réagir avec un (poly)isocyanate de la manière décrite plus haut. Comme composés X de ce type, on peut utiliser ceux à fonction OH, issus de la réaction d'un composé à fonction(s) époxy avec un phosphate de formule (2) ainsi que ceux issus de la réaction d'un composé à fonction époxy avec un polyaminoéther de formule (3) ou de formule (4) qui ont été décrits plus haut. Dans ce dernier cas, on obtient une composition qui est soit auto émulsifiable soit, si elle ne l'est pas à un degré suffisant, qui peut l'être rendue par addition supplémentaire à la composition d'un additif hydrophile non réactif du type qui a été décrit ci-dessus.

Selon une autre variante de .ce second mode de réalisation, l'additif greffé peut être apporté par le (poly) isocyanate lui-même. Comme additifs greffables sur les (poly)isocyanates, on peut citer les additifs hydrophiles mentionnés dans le brevet US 4663377 à l'enseignement duquel on pourra se référer. On peut faire réagir le (poly)isocyanate ainsi traités avec un composé X hydrophobe et dans ce cas on obtient là encore une composition qui est soit auto émulsifiable soit, si elle ne l'est pas à un degré suffisant, qui peut l'être rendue par addition d'un additif hydrophile du type non réactif comme décrit précédemment.

Enfin, il est aussi possible de faire réagir un (poly)isocyanate greffé avec l'additif hydrophile avec un composé X lui-même hydrophile pour obtenir de même une composition auto émulsifiable ou dont on peut aussi améliorer la propriété autoémulsification de la manière décrite ci-dessus.

L'invention couvre aussi un procédé de préparation d'un revêtement sur un substrat qui est caractérisé en ce qu'on mélange une composition du type durcisseur, comme décrite plus haut, avec au moins un composé porteur d'au moins une fonction à hydrogène mobile choisie parmi les fonctions hydroxyle, amine primaire, amine secondaire et la fonction SH ou avec un composé contenant des fonctions précurseurs susceptibles de libérer des fonctions hydroxyles; et on applique le mélange obtenu sur le substrat. La réticulation se déroule ensuite.

Il s'agit en fait d'un procédé de préparation d'un revêtement à base de polyuréthane par réaction d'un durcisseur avec une résine, procédé bien connu de l'homme du métier.

Il est à noter que la réticulation du composé durcisseur de l'invention peut aussi se faire sur lui-même par

l'action de l'eau de l'atmosphère.

Le mélange précité de la composition du type durcisseur et du composé porteur d'au moins une fonction à hydrogène mobile se fait en présence d'un solvant et aussi généralement et d'une manière connue de l'homme du métier en présence d'additifs organiques ou inorganiques, comme un additif de rhéologie, un solvant, un épaississant, des agents de surface, des catalyseurs, en fonction des propriétés recherchées.

On peut aussi, lors de la formation du mélange, ajouter d'autres composés comme des résines aminoplastiques (résine « aminoplast ») ou époxy.

De préférence, les composés sont choisis parmi les polyols qui peuvent être utilisés seuls ou en mélange. Ce peut être avantageusement des polymères acryliques, polyesters, polyuréthanes ou des hybrides de ces polymères. On peut mentionner aussi les polyéthers qui ne sont pas préférés toutefois.

Comme fonctions précurseurs susceptibles de libérer des fonctions hydroxyles, on peut citer les fonctions époxy, carbonates ou dioxolane. Ces fonctions précurseurs libèrent les fonctions hydroxy par réaction avec un nucléophile adéquat comme une amine ou de l'eau, éventuellement en présence d'un catalyseur qui peut être un composé acide ou un acide de Lewis dans une quantité en poids qui peut être comprise par exemple entre 50 et 5000 ppm, plus particulièrement entre 100 et 500 ppm, quantité exprimée en poids de catalyseur par rapport à l'extrait sec de la composition du type durcisseur et du composé porteur d'au moins une fonction à hydrogène mobile.

Une fois le mélange précité déposé, la réaction entre la composition du type durcisseur et le composé porteur d'au moins une fonction à hydrogène mobile peut se faire à température ambiante ou, plus généralement à chaud à une température qui peut être comprise entre 30°C et 300°C, de préférence entre 40°C et 250°C et encore plus préférentiellement entre 50°C et 150°C. La température et le temps de réticulation sont adaptés en fonction du substrat. Dans le cas de substrats sensibles à la température on utilisera plus particulièrement des catalyseurs de réticulation.

Le substrat peut être un substrat métallique par exemple en aluminium ou acier, en particulier en acier inoxydable. Ce peut être aussi un substrat en polymère plastique. Le substrat peut être un élément de carrosserie automobile par exemple. Le substrat peut aussi être un substrat en bois ou en papier. Par ailleurs, le substrat peut déjà comporter une couche protectrice du type peinture ou vernis.

Le composé de l'invention a des propriétés d'accélération de séchage.

C'est pourquoi, l'invention couvre aussi l'utilisation de ce composé comme accélérateur de séchage dans un procédé de préparation d'un revêtement sur un substrat, procédé dans lequel on mélange une composition de type durcisseur comprenant ledit composé avec un composé porteur d'au moins une fonction à hydrogène mobile choisie parmi les fonctions hydroxyle, amine primaire, amine secondaire et la fonction SH et où l'on dépose le mélange ainsi obtenu sur le substrat.

Ce qui a été dit plus haut au sujet du composé porteur d'au moins une fonction à hydrogène mobile, du procédé de préparation du revêtement et du substrat s'applique bien entendu ici.

En outre, on notera que le composé de l'invention, outre la propriété d'accélération de séchage, permet d'obtenir plus rapidement d'autres propriétés comme la dureté et la résistance chimique. Il peut ainsi offrir un bon compromis de propriétés, notamment il peut présenter aussi une durée de vie en pot (pot life) améliorée.

Des exemples vont maintenant être donnés.

Les produits utilisés dans ces exemples sont les suivants :

- TOLONATE HDT LV2: polyisocyanate à base HDI sur base isocyanurate de basse viscosité vendu par la société RHODIA, de teneur en fonction isocyanate de l'ordre de 22,5 % et de viscosité d'environ 600 +/- 150 mPa.s à 25°C, fonctionnalité en fonction isocyanate de l'ordre de 3,3
- TOLONATE HDT: polyisocyanate à base HDI sur base isocyanurate vendu par la société RHODIA, de teneur en fonction isocyanate de l'ordre de 22 % et de viscosité de 2400 + /- 400 mPa.s à 25°C, fonctionnalité en fonction isocyanate de l'ordre de 3,6
- TOLONATE XFD 90 B : polyisocyanate à base HDI sur base isocyanurate vendu par la société RHODIA, de teneur en fonction isocyanate de l'ordre de 17,4 % environ et de viscosité de 2000 + /- 1000 mPa.s à 25°C
- TOLONATE HDB : polyisocyanate à base HDI sur base biuret vendu par la société RHODIA, de teneur en fonction isocyanate de 22% en poids, de viscosité de 9000 +/- 2000 mPas à 25° et de fonctionnalité 3,7.
- TOLONATE HDB LV : polyisocyanate à base HDI sur base biuret de basse viscosité vendu par la société RHODIA, de teneur en fonction isocyanate de l'ordre de 23,5 %, de viscosité de 2000 +/- 500 mPa.s à 25°C, de fonctionnalité en fonction isocyanate de l'ordre de 3,5
- Bisphénol A hydrogéné : 2,2 Bis (4 hydroxy cyclohexyl) propane (HBPA) encore appelé 4,4' isopropylidène dicyclohexanol (RN Cas : 80-04-6), poids moléculaire 240g, produit solide fourni par la société MARUZEN constitué de deux cycles reliés entre eux par une chaîne aliphatique ramifiée de 3 carbones et possédant deux fonctions hydroxyles secondaire. La fonctionnalité OH est de 2.
- Tricyclodécane di méthanol (TCDM) : mélange liquide visqueux d'isomères vendu par la société CELANESE, dont les composés sont constitués de 3 cycles accolés et de deux fonctions hydroxyles primaires. La fonctionnalité OH

est de 2.

- RHODIANTAL : Isobornyl cyclohexanol (IBCH). Mélange liquide visqueux d'isomères vendu par la société RHODIA dont les composés sont constitués de 3 cycles dont 2 accolés porteurs de chaînes aliphatiques à 1 carbone et 1 cycle relié aux deux autres par une simple liaison, ce cycle étant porteur d'une seule fonction hydroxyle secondaire. Le poids moléculaire moyen étant de 236,4g. L'indice d'hydroxyle est compris entre 215 et 235 mg KOH /g.
- Acétate de n butyle : AcO n Butyle
- JONCRYL SCX 922 : polyol acrylique à 80% d'extrait sec et à 4,2% en OH
- SYNOCURE 852 BA 80 : polyol acrylique à 80% d'extrait sec et à 4,1% en OH
- DBDL : dibutyl-dilaurate d'étain

EXEMPLE 1

Cet exemple concerne la préparation d'un composé selon l'invention à fonctionnalité isocyanate et comportant en outre de fonctions uréthannes.

Dans un réacteur on charge successivement sous courant d'azote 450,5 g de TOLONATE HDT LV2, 48,5 g de HBPA (0,203mole) et 111,8 g d'acétate de n butyle. Le titre NCO du TOLONATE HDT LV2 de départ est de 0,537 mole pour 100 g. Le ratio HBPA / (HBPA + isocyanate de départ) est de 9,72 %.

Le mélange est chauffé à 120°C pendant 3 heures. Le titre NCO passe de 0,537 mole pour 100 g pour le TOLONATE HDT LV2 de départ à 0,331 pour le mélange réactionnel en fin de réaction.

On récupère 610,8 g de formulation de composé selon l'invention à 82 % d'extrait sec dans l'acétate de n butyle.

Le titre NCO de la formulation de composé selon l'invention est de 0,33 mole pour 100 g. Sa viscosité est de 1305 mPa.s à 25°C pour un extrait sec de 82 % dans l'acétate de n butyle.

Le produit est séparé par chromatographie par perméation de gel (GPC) sur une colonne polymer gel en utilisant comme solvant d'élution le dichlorométhane et analysé par infra rouge.

Le taux en monomère HDI résiduel donné par GPC est de 0,48%.

La fonctionnalité en fonctions NCO est de 4.

EXEMPLES 2 et 3

Ces exemples concernent la préparation de composés selon l'invention à fonctionnalité isocyanate et comportant en outre de fonctions uréthannes.

On procède comme pour l'exemple 1 mais on fait varier les conditions opératoires et le ratio HBPA/ (polyisocyanate de départ +HBPA). Les résultats sont présentés dans le tableau 1 ci-après.

Tableau 1

| Ex | Q1 (g) | Q2 (g) | R % | T /t | ES (%) | Ti | Viscosité à 25°C en mPa.s | Ta |
|---|---|---|---|---|---|---|---|---|
| 2 | 1042 | 76,8 | 6,9 | 120°C 30 mn + 100°C/ 2H | 100 | 0,466 | 8135 | 0,2 |
| 3 | 432,7 | 47,5 | 9,9 | 130°C 30 mn + 1 H 120°C + 3H15100°C | 85,7 Ac O n Butyle | 0,356 | 2079 | 0,28 |

Q1 : Quantité de TOLONATE HDT LV2
Q2 : Quantité de HBPA
R : Ratio pondéral HBPA/ (HBPA+ HDT LV2)
T : Température de réaction ; t : temps de réaction
ES : extrait sec
Ti : Titre NCO en mole pour 100g
Ta : Taux de monomère HDI résiduel

EXEMPLE 4

Cet exemple concerne la préparation d'un composé selon l'invention à fonctionnalité isocyanate et comportant des fonctions allophanates

Dans un réacteur on charge successivement sous courant d'azote 372,4 g de TOLONATE HDT LV2, de 27,4

g d'HBPA (0,114mole). On ajoute 200 ppm de dibutyl dilaurate d'étain par rapport au TOLONATE HDT LV2. Le titre NCO du TOLONATE HDT LV2 de départ est de 0,55 mole pour 100 g. Le ratio pondéral HBPA/(HBPA + isocyanate de départ) est de 6,85 %.

Le mélange est chauffé à 110°C pendant 48h. Le titre NCO du mélange réactionnel en fin de réaction est de 0,377 mole pour 100g.

On récupère 400g.

Le titre NCO de la formulation de composé selon l'invention à fonctions allophanates est de 0,377 mole pour 100g. Sa viscosité est de 14 000 mPa.s à 25 °C et à 90 % d'extrait sec dans l'acétate de n butyle et 800 mPa.s à 25°C pour un extrait sec de 70% dans l'acétate de n butyle.

Le produit est séparé par chromatographie par GPC sur une colonne polymer gel en utilisant comme solvant d'élution le dichlorométhane et analysé par infra rouge.

Le taux en monomère HDI résiduel donné par GPC est de 0,5 %.

La fonctionnalité en fonctions NCO est de 8.

EXEMPLE 5

Cet exemple concerne la préparation d'un composé selon l'invention à fonctionnalité isocyanate et comportant des fonctions uréthannes.

On procède comme pour l'exemple 1 mais en utilisant l'IBCH en lieu et place de l'HBPA.

Dans un réacteur on charge successivement sous courant d'azote 459 g de TOLONATE HDT LV2, 46,3 g d'IBCH (0,196 mole). Le titre NCO du TOLONATE HDT LV2 de départ est de 0,55 mole pour 100 g. Le ratio pondéral IBCH./ (IBCH + isocyanate de départ) est de 9,2 %.

Le mélange est chauffé à 110°C pendant 7 heures 30. Le titre NCO passe de 0,496 mole pour 100 g pour le milieu réactionnel de départ à 0,466 mole pour 100 g en fin de réaction.

On récupère 482 g de produit.

Le titre NCO de la formulation de composé selon l'invention à fonction uréthanne est de 0,466 mole pour 100 g. Sa viscosité est de 2057 mPa.s à 25°C.

Le produit est séparé par chromatographie par GPC sur une colonne polymer gel en utilisant comme solvant d'élution le dichlorométhane et analysé par infra rouge.

Le taux en monomère HDI. résiduel donné par GPC est de 0,2%.

La fonctionnalité en fonctions NCO est de 3.

EXEMPLE 6

Cet exemple concerne la préparation d'un composé selon l'invention à fonctionnalité isocyanate et comportant des fonctions uréthannes.

On procède comme pour l'exemple 5 mais en utilisant le TOLONATE HDT à la place du TOLONATE HDT LV2.

Dans un réacteur on charge successivement sous courant d'azote 374,4 g de TOLONATE HDT (1,95 mole de NCO), 38,1 g d'IBCH (0,16 mole). Le titre NCO du TOLONATE HDT de départ est de 0,52 mole pour 100 g. Le ratio pondéral IBCH / (IBCH + isocyanate de départ) est de 9,2 %.

Le mélange est chauffé à 110°C pendant 6 heures 15. Le titre NCO passe de 0,471 mole pour 100 g pour le milieu réactionnel de départ à 0,435 mole pour 100 g en fin de réaction.

On récupère 389,7 g de produit.

Le titre NCO de la formulation de composé selon l'invention à fonction uréthanne est de 0,435 mole pour 100g. Sa viscosité est de 8498 mPa.s à 25°C pour un extrait sec de 100%.

Le produit est séparé par chromatographie par GPC sur une colonne polymer gel en utilisant comme solvant d'élution le dichlorométhane et analysé par infra rouge.

Le taux en monomère HDI résiduel donné par GPC est de 0,2%.

La fonctionnalité en fonctions NCO est de 3,5.

EXEMPLE 7

Cet exemple concerne la préparation d'un composé selon l'invention à fonctionnalité isocyanate èt comportant des fonctions uréthanne.

On procède comme pour l'exemple 1 mais en utilisant le Tricyclodécane diméthanol (TCDM) en lieu et place de l'HBPA.

Dans un réacteur on charge successivement sous courant d'azote 300 g de TOLONATE HDT LV2, 21,6 g de TCDM (0,11 mole). Le titre NCO du TOLONATE HDT LV2 de départ est de 0,55 mole pour 100 g. Le ratio TCDM /

(TCDM + isocyanate de départ) est de 6,7 %.

Le mélange est chauffé à 80°C pendant 2H. Le pourcentage de fonctions isocyanates consommé est de 13,3 %.

On récupère 320 g de produit.

Le titre NCO de la formulation de composé selon l'invention à fonctions uréthannes est de 0,444 mole pour 100g. Sa viscosité est de 1220 mPa.s à 25°C.

EXEMPLE 8

Cet exemple concerne la préparation d'un composé selon l'invention à fonctionnalité isocyanate et comportant des fonctions uréthannes et biuret.

Dans un réacteur tricol, on charge successivement 271,2 g de de n butyle acétate, 101g de HBPA, 0,1g de triméthylolpropane, 0,1 g de 1,6 Hexanediol, 0,1 g d'anhydride phtalique et 0,1g d'anhydride maléique. Le milieu réactionnel agité est chauffé à une température d'environ 130°C. A 125°C l'ensemble des réactifs est solubilisé. On procède à une distillation azéotropique pour éliminer l'eau contenue dans les réactifs et l'eau de condensation de la réaction d'estérification. Après 1 H 30 de réaction, la température du milieu réactionnel est abaissée à 110°C et on ajoute alors 983,4g de TOLONATE HDB dont le titre NCO mesuré est de 0,527 moles de fonctions NCO pour 100g. Le nombre total de moles de fonctions isocyanates est donc de 5,182 moles, le nombre total de moles de fonctions hydroxyles est de 0,76. Le ratio molaire des fonctions hydroxyles par rapport aux fonctions isocyanates est de 14,66 %. Le ratio pondéral HBPA /HDB est de 10,27%.

Le titre NCO du milieu réactionnel est suivi régulièrement. Il est de 0,331 moles de NCO pour 100g après 2H20, 0,327 après 2H 20 et de 0,326 après 4H 30 de réaction.

On arrête la réaction et on ajuste l'extrait sec à 80% poids par ajout d'acétate de n butyle.

Le produit fini présente les caractéristiques suivantes :

- Titre NCO = 13,69% poids
- - Viscosité = 9140 mPas à 25°C mesuré par la méthode dite à la chute de bille
- Le point éclair de la formulation est de 48°C.

La formulation contient les composés isocyanates majoritaires suivants

- 20% poids d'acétate de n butyle
- des oligomères polyisocyanates sur base biuret de HDI
  - ○ 17 % poids de biuret vrai de HDI (3 fonctions isocyanates, 3 chaînes hexaméthylène et une fonction biuret)
  - ○ 8% poids de bis biuret de HDI (4 fonctions isocyanates, 5 chaînes hexaméthylènes et deux fonctions biuret)
  - ○ 1,5 % de dimère de HDI vrai (2 fonctions isocyanates, 2 chaînes hexaméthylènes et une fonction uretidine dione)
  - ○ 0,24 % poids de HDI monomère
- des oligomères polyisocyanates sur base biuret de HDI plus lourds que le bis biuret
- et des oligomères polyisocyanates sur base biuret de HDI et possédant des fonctions uréthanes du HBPA

Les deux derniers points représentent le complément à 100 % poids soit 53,26% poids.

La fonctionnalité en fonctions isocyanates est au moins égale à 4,5.

EXEMPLE 9

On procède comme pour l'exemple 8 sauf que les quantités de matière de départ sont les suivantes :

| | |
|---|---|
| N butyle acétate | 224,8 g |
| TOLONATE HDB | 800,2 g |
| HBPA | 98,5 g |
| triméthylolpropane | 0,1g |
| 1,6 Hexanediol, | 0,1g |
| anhydride phtalique | 0,1g |

(suite)

| | |
|---|---|
| anhydride maléique | 0,1g |
| Ratio molaire OH / NCO | 18% |
| Ratio pondéral HBPA / HDB | 12,37% |

Le produit fini présente les caractéristiques suivantes :

- Titre NCO = 12,39% poids
- Viscosité = 18664 mPas à 25°C mesuré par la méthode dite à la chute de bille
- La couleur exprimée en hazen est de 60.
- Extrait sec 79,6%

La formulation contient les composés isocyanates majoritaires suivants

- 20,4% poids d'acétate de n butyle
- des oligomères polyisocyanates sur base biuret de HDI
    - ○ 14,1 % poids de biuret vrai de HDI (3 fonctions isocyanates, 3 chaînes hexaméthylène et une fonction biuret)
    - ○ 6,7% poids de bis biuret de HDI (4 fonctions isocyanates, 5 chaînes hexaméthylènes et deux fonctions biuret)
    - ○ 1,2 % de dimère de HDI vrai (2 fonctions isocyanates, 2 chaînes hexaméthylènes et une fonction uretidine dione)
    - ○ 0,27 % poids de HDI monomère
- des oligomères polyisocyanates sur base biuret de HDI plus lourds que le bis biuret
- et des oligomères polyisocyanates sur base biuret de HDI et possédant des fonctions uréthanes du HBPA

Les deux derniers points représentent le complément à 100 % poids soit 57,33%

La fonctionnalité en fonctions isocyanates est au moins égale à 5.

EXEMPLE 10

On procède comme pour l'exemple 8 sauf que les quantités de matière de départ sont les suivantes :

| | |
|---|---|
| N butyle acétate | 400g |
| TOLONATE HDB | 967,2g |
| HBPA | 149g |
| triméthylolpropane | 0,14g |
| 1,6 Hexanediol, | 0,14g |
| anhydride phtalique | 0,14g |
| anhydride maléique | 0,14g |
| Ratio molaire OH / NCO | 22,23 % |
| Ratio pondéral HBPA / HDB | 15,4% |

Le produit fini présente les caractéristiques suivantes :

- Titre NCO = 10,5% poids
- Viscosité = 12273 mPas à 25°C mesuré par la méthode dite à la chute de bille
- La couleur exprimée en hazen est de 60.
- Extrait sec 73,6%

La fonctionnalité en fonctions isocyanates est au moins égale à 5.

EXEMPLE 11

On procède comme pour l'exemple 8 sauf que les quantités de matière de départ sont les suivantes :

| | |
|---|---|
| N butyle acétate | 400g |
| TOLONATE HDB | 726,1g |
| HBPA | 149g |
| triméthylolpropane | 0,14g |
| 1,6 Hexanediol, | 0,14g |
| anhydride phtalique | 0,14g |
| anhydride maléique | 0,14g |
| Ratio molaire OH / NCO | 29,6 % |
| Ratio pondéral HBPA / HDB | 20,5% |

Le produit fini présente les caractéristiques suivantes :

- Titre NCO = 8,19% poids
- Viscosité = 46953 mPas à 25°C mesuré par la méthode dite à la chute de bille
- La couleur exprimée en hazen est de 60.
- Extrait sec 68,6%

La fonctionnalité en fonctions isocyanates est au moins égale à 5.

EXEMPLES 12 à 17: Compositions comprenant les composés à fonctionnalité isocyanates de l'invention

Les compositions sont préparées à partir d'un composé à fonctionnalité isocyanate de l'invention et d'un autre polyisocyanate. L'extrait sec des formulations est de 80%. Le solvant est l'acétate de n butyle.

La nature et la quantité des constituants de la composition sont indiquées dans le tableau ci-dessous.

| Composition | Composé à fonctionnalité isocyanate de l'invention (quantité en g) | Autre polyisocyanate (quantité en g) |
|---|---|---|
| Exemple 12 | Exemple 10 (51,32) | Tolonate HDB (16,4) |
| Exemple 13 | Exemple 10 (50) | Tolonate HDT (16,1) |
| Exemple 14 | Exemple 10 (50,8) | Tolonate HDB LV (16,21) |
| Exemple 15 | Exemple 10 (50,25) | Tolonate HDT LV2 (16,08) |
| Exemple 16 | Exemple 11 (36,4) | Tolonate HDB (20,86) |
| Exemple 17 | Exemple 11 (34,7) | Tolonate HDB LV (19,7) |

Les caractéristiques des compositions sont indiquées dans le tableau ci-dessous:

| Composition | Titre NCO en mole NCO/100 g | Viscosité en mPa à 25°C |
|---|---|---|
| Exemple 12 | 0,31 | 11746 |
| Exemple 13 | 0,312 | 9397 |
| Exemple 14 | 0,324 | 8571 |
| Exemple 15 | 0,323 | 6877 |

(suite)

| Composition | Titre NCO en mole NCO/100 g | Viscosité en mPa à 25°C |
|---|---|---|
| Exemple 16 | 0,323 | 25875 |
| Exemple 17 | 0,327 | 16550 |

Les exemples qui suivent illustrent l'utilisation de compositions durcisseurs comparative et selon l'invention dans des applications de type vernis. Ces exemples font référence à des tests qui sont décrits ci-dessous.

Pot life : la valeur du « pot life » (durée de vie en pot) s'obtient grâce à la mesure de la viscosité à la coupe DIN 4. La valeur du pot life correspond au temps nécessaire au doublement de cette viscosité.

Temps de séchage : le temps de séchage est déterminé selon la méthode allemande DIN 53150. On indique ci-dessous les temps T2 et T3 qui ont été mesurés selon cette norme. T2 correspond au temps au bout duquel un papier ne colle plus à la surface du film de vernis, après avoir subi la pression d'un poids de 20 g durant 1 minute environ. T3 est déterminé de la même manière que T2 avec un poids de 200 g.

Brillance : cette mesure est caractéristique de l'homogénéité et de l'aspect des films. Elle est faite après 7 jours de séchage dans une salle conditionnée à 20°C à l'aide d'un brillancemètre Erichsen modèle S40.

Dureté Persoz : les mesures de dureté Persoz se font dans une salle conditionnée à 23±3°C et une humidité relative de 50±10%. L'appareil utilisé est un pendule d'essai type 300 de chez Erichsen avec butée de lancement et comptage automatique. Le principe du pendule de dureté se base sur les oscillations d'un pendule placé sur le film. Le nombre d'oscillations est d'autant plus grand que le vernis est dur et sec. La mesure de reprise de dureté est le nombre d'oscillations que met le pendule à s'amortir puis s'arrêter. La durée d'une oscillation est de une seconde. L'essai est fini lorsque l'amortisseur des oscillations atteint une amplitude de 4°. Les mesures sont effectuées régulièrement au cours du séchage, à 1, 3 et 7 jours.

Test méthyléthylcétone : ce test caractérise la résistance aux solvants, ici la méthyléthylcétone. Le test est effectué après 7 jours.

Résistance au choc : ce test permet de mettre en évidence le caractère fragile d'un revêtement. L'essai consiste à soumettre le revêtement après 7 jours de séchage au choc d'un percuteur de dimensions et de poids déterminés dont la hauteur de chute est réglable. On détermine la hauteur minimale à partir de laquelle le feuil de peinture ou de vernis ne présente plus de fissurations ou d'écaillages. La valeur est fixée à 100 cm pour la norme Afnor et à 80 cm pour la norme ASTM.

EXEMPLES 18 à 20

Ces exemples concernent l'utilisation de compositions durcisseurs comparative et selon l'invention dans une application de type vernis 2K en phase solvant pour la réparation automobile.

On donne dans le tableau 2 ci-dessous les caractéristiques des compositions durcisseurs qui sont constituées d'une partie A à base d'un polyol et d'une partie B à base de composés à fonctionnalité isocyanate comparatif ou selon l'invention.

Dans le cas de l'exemple 18, le composé comparatif à fonctionnalité isocyanate est le TOLONATE XFD qui est un isocyanate aliphatique de haute fonctionnalité possédant de bonnes propriétés de séchage. Les exemples 19 et 20 comportent comme composés à fonctionnalité isocyanate les composés des exemples 2 et 1 ci-dessus respectivement.

Tableau 2

| | Exemple 18 comparatif | Exemple 19 | Exemple 20 |
|---|---|---|---|
| Partie A | | | |
| JONCRYL SCX 922 | 78,00 | 78,00 | 76,00 |
| DBDL Fluka (1% dans acétate de butyle) | 1 | 1 | 1 |
| Acétate de butyle | 45 | 41,9 | 44 |
| | | | |

(suite)

| | | | |
|---|---|---|---|
| Partie B | | | |
| TOLONATE XFD | 41,20 | | |
| Composé de l'exemple 2 | | 36,80 | |
| Composé de l'exemple 1 | | | 50,00 |

La réticulation est effectuée à 23°C avec 55% d'humidité relative. La viscosité à la coupe DIN 4 est ajustée à 23 s.

On donne ci-dessous les propriétés des vernis obtenus mesurées selon différents tests.

Pot life : les résultats de ce test sont donnés dans le tableau 3 qui suit.

Tableau 3

| | Exemple 18 comparatif | Exemple 19 | Exemple 20 |
|---|---|---|---|
| Durée (min) | 40 | 42 | 40 |

On note donc des pot life similaires pour les trois exemples.

Temps de séchage : les résultats de ce test sont donnés dans le tableau 4 qui suit.

Tableau 4

| | Exemple 18 comparatif | Exemple 19 | Exemple 20 |
|---|---|---|---|
| T2 (min) | 200 | 166 | 133 |
| T3 (min) | 230 | 175 | 171 |

On note une diminution importante des temps de séchage T2 et T3 pour les compositions selon l'invention.

Brillance : les résultats de ce test sont donnés dans le tableau 5 qui suit.

Tableau 5

| Exemple 18 comparatif | Exemple 19 | Exemple 20 |
|---|---|---|
| 94 | 94 | 94 |

Toutes les compositions présentent un aspect satisfaisant.

Dureté Persoz : les résultats de ce test sont donnés dans le tableau 6 qui suit.

Tableau 6

| Temps | Exemple 18 comparatif | Exemple 19 | Exemple 20 |
|---|---|---|---|
| 1 jour | 72 | 67 | 102 |
| 3 jours | 67 | 81 | 100 |
| 7 jours | 102 | 125 | 139 |

Test méthyléthylcétone : les résultats de ce test sont donnés dans le tableau 7 qui suit.

Tableau 7

| Exemple 18 comparatif | Exemple 19 | Exemple 20 |
|---|---|---|
| 72 | 86 | 120 |

On note une amélioration de la résistance aux solvants pour les compositions de l'invention.

Résistance au choc : tous les vernis obtenus à partir des compositions des exemples ci-dessus passent le test.

En conclusion, on voit donc à partir des tests donnés ci-dessus que les compositions de l'invention apportent

une nette amélioration du temps de séchage et on constate que les autres propriétés du film ne sont pas altérées et peuvent même être améliorées.

EXEMPLES 21 à 23

Ces exemples concernent l'utilisation de compositions durcisseurs comparative et selon l'invention dans une application de type vernis 2K en phase solvant pour l'industrie générale.

On donne dans le tableau 8 ci-dessous les caractéristiques des compositions durcisseurs qui sont constituées d'une partie A à base d'un polyol et d'une partie B à base de composés à fonctionnalité isocyanate comparatif ou selon l'invention.

Dans le cas de l'exemple 21, le composé comparatif à fonctionnalité isocyanate est le TOLONATE XFD mentionné plus haut. Les exemples 22 et 23 comportent comme composés à fonctionnalité isocyanate les composés des exemples 2 et 3 ci-dessus respectivement.

Tableau 8

| | Exemple 21 comparatif | Exemple 22 | Exemple 23 |
|---|---|---|---|
| Partie A | | | |
| SYNOCURE 852 BA 80 | 78,00 | 78,00 | 76,00 |
| DBDL Fluka (1% dans acétate de butyle) | 1 | 1 | 1 |
| Acétate de butyle | 41 | 43,4 | 46,7 |
| | | | |
| Partie B | | | |
| TOLONATE XFD | 41,20 | | |
| Composé de l'exemple 2 | | 36,80 | |
| Composé de l'exemple 3 | | | 46,50 |

La réticulation est effectuée à 23°C avec 55% d'humidité relative. La viscosité à la coupe DIN 4 est ajustée à 23 s.

On donne ci-dessous les propriétés des vernis obtenus mesurées selon différents tests.

Pot life : les résultats de ce test sont donnés dans le tableau 9 qui suit.

Tableau 9

| | Exemple 21 comparatif | Exemple 22 | Exemple 23 |
|---|---|---|---|
| Durée (min) | 35 | 60 | 70 |

On note une amélioration nette dans les cas des compositions selon l'invention.

Temps de séchage : les résultats de ce test sont donnés dans le tableau 10 qui suit.

Tableau 10

| | Exemple 21 comparatif | Exemple 22 | Exemple 23 |
|---|---|---|---|
| T2 (min) | 450 | 385 | 330 |
| T3 (min) | 545 | 530 | 435 |

On note une diminution importante des temps de séchage T2 et T3 pour les compositions selon l'invention.

Brillance : les résultats de ce test (effectué ici après 3 jours de séchage) sont donnés dans le tableau qui suit.

Tableau 11

| Exemple 21 comparatif | Exemple 22 | Exemple 23 |
|---|---|---|
| 97 | 96 | 98 |

Toutes les compositions présentent un aspect satisfaisant.

Dureté Persoz : les résultats de ce test sont donnés dans le tableau 12 qui suit.

Tableau 12

| Temps | Exemple 21 comparatif | Exemple 22 | Exemple 23 |
|---|---|---|---|
| 1 jour | 150 | 167 | 205 |
| 3 jours | 197 | 215 | 245 |
| 7 jours | 252 | 256 | 303 |

Les valeurs obtenues pour les compositions de l'invention sont supérieures tout au long de formation du film, tout particulièrement dans le cas de l'exemple 23.

On note là encore en conclusion que les compositions de l'invention apportent une amélioration du temps de séchage sans altération des autres propriétés.

EXEMPLES 24 et 25

Ces exemples concernent l'utilisation de compositions durcisseurs comparative et selon l'invention dans une application de type vernis 2K en phase solvant pour l'industrie générale.

On donne dans le tableau 13 ci-dessous les caractéristiques des compositions durcisseurs qui sont constituées d'une partie A à base d'un polyol et d'une partie B à base de composés à fonctionnalité isocyanate comparatif ou selon l'invention.

Dans le cas de l'exemple 24 le composé à fonctionnalité isocyanate utilisé est celui de l'exemple 3 Le composé à fonctionnalité isocyanate utilisé dans l'exemple comparatif 25 est le TOLONATE XFD mentionné plus haut.

Les conditions de réticulation et de séchage sont modifiées. La réticulation est conduite avec 10' de désolvatation à température ambiante (flash off) et 30' à 60°C. On n'utilise pas de catalyseur.

Tableau 13

| | Exemple 24 | Exemple 25 comparatif |
|---|---|---|
| Partie A | | |
| SYNOCURE 852 BA 80 | 76,00 | 78,00 |
| DBDL FLuka (1% dans acétate de butyle) | 2 | 2 |
| Acétate de butyle | 43,3 | 41,4 |
| | | |
| Partie B | | |
| Composé de l'exemple 3 | 46,50 | |
| TOLONATE XFD | | 40,50 |

On donne dans le tableau récapitulatif 14 ci-dessous les résultats des différents tests conduits sur les vernis obtenus.

Tableau 14

| | Dureté Persoz 1 Jour | Dureté Persoz 7 Jours | Brillance | Choc Afnor | Test méthyléthylcétone |
|---|---|---|---|---|---|
| Exemple 25 comparatif | 48 | 301 | 96 | 100 | 90 |
| Exemple 24 | 65 | 313 | 97 | 100 | 182 |

EXEMPLES 26 et 27

Ces exemples concernent l'utilisation de compositions durcisseurs comparative et selon l'invention dans une application de type vernis 2K en phase solvant pour l'industrie générale.

Le composé à fonctionnalité isocyanate utilisé dans l'exemple comparatif 26 est un mélange à base HDI et d'IPDI sous forme trimère dans un rapport respectif en poids de 60/40. Dans le cas de l'exemple 27 le composé à fonctionnalité isocyanate utilisé est celui de l'exemple 3.

La réticulation est effectuée à 23°C avec 55% d'humidité relative.

Le tableau suivant 15 regroupe les caractéristiques de ces différentes compositions durcisseurs.

Tableau 15

| | Exemple comparatif 26 | Exemple 27 |
|---|---|---|
| NCO composé à fonctionnalité isocyanate (tel quel) | 17,27 % | 14,9 % |
| extrait sec composé à fonctionnalité isocyanate | 85,4 % | 100 % |

On donne ci-dessous les résultats des différents tests conduits sur les vernis obtenus.

<u>Brillance :</u>

Il n'y a pas de différence entre les compositions évaluées et toutes présentent un aspect satisfaisant (brillance 20° > 97)

<u>Dureté Persoz</u> :

Tableau 16

| | Exemple comparatif 26 | Exemple 27 |
|---|---|---|
| 1 jour | 110 | 100 |
| 3 jours | 194 | 181 |

<u>Résistance au choc :</u>

Tableau 17

| | Exemple comparatif 26 | Exemple 27 |
|---|---|---|
| Afnor J7 | 40 cm | 100 cm |
| ASTM J7 | 35 cm | 80 cm |

La différence est très nette pour le mélange de l'exemple comparatif qui est très cassant par rapport au mélange selon l'exemple de l'invention.

EXEMPLES 28 à 30

Ces exemples concernent l'utilisation de compositions durcisseurs comparative et selon l'invention dans une application de type vernis 2K en phase solvant pour l'industrie générale. On donne dans le tableau 18 ci-dessous les caractéristiques des compositions durcisseurs qui sont constituées d'une partie A à base d'un polyol et d'une partie B à base de composés à fonctionnalité isocyanate comparatif ou selon l'invention.

Ces exemples mettent en évidence l'importance de la réaction préalable du polyisocyanate avec le composé à hydrogène labile par rapport au simple ajout physique de ce dernier dans la partie A.

Dans le cas de l'exemple 28, le composé à fonctionnalité isocyanate utilisé est celui de l'exemple 3. Dans le

cas de l'exemple 29, le composé comparatif à fonctionnalité isocyanate est le TOLONATE XFD 90 B qui est un isocyanate aliphatique de haute fonctionnalité possédant de bonnes propriétés de séchage. Dans le cas de l'exemple 30, le composé comparatif est le TOLONATE HDT LV2 et dans ce cas, la partie A est additionnée avec une quantité d'HBPA égale à la quantité utilisée pour synthétiser le composé de l'exemple 3.

La réticulation est effectuée à 23°C avec 55% d'humidité relative.

Le tableau suivant 18 regroupe les caractéristiques de ces différentes compositions durcisseurs.

Tableau 18

| | Exemple 28 | Exemple 29 comparatif | Exemple 30 comparatif |
|---|---|---|---|
| **Partie A** | | | |
| SYNOCURE 852BA80 | 74,22 | 70,91 | 68,78 |
| HBPA | | | 2,99 |
| DBDL FLuka (1% dans AcBu) | 2,00 | 1,82 | 1,77 |
| Acétate de butyle | 23,79 | 27,27 | 26,46 |
| | | | |
| **Partie B** | | | |
| Composé de l'exemple 3 | 43,00 | | |
| TOLONATE XFD | | 36,00 | |
| TOLONATE HDT LV2 | | | 31,90 |
| Acétate de butyle | 5,00 | 5,00 | 5,00 |

On donne ci-dessous les résultats des différents tests conduits sur les vernis obtenus.

Pot life :

Tableau 19

| | Exemple 28 | Exemple 29 comparatif | Exemple 30 comparatif |
|---|---|---|---|
| Durée (min) | 55 | 40 | 40 |

Le pot life obtenu pour le mélange de l'exemple 28 est meilleur que dans le cas des exemples comparatifs 29 et 30.

Temps de séchage :

Tableau 20

| | Exemple 28 | Exemple 29 comparatif | Exemple 30 comparatif |
|---|---|---|---|
| T1 (min) | 60 | 75 | 280 |

L'addition de l'HBPA avec la partie A se traduit par un temps de séchage hors poussière très élevé comparé au composé de l'invention. La réaction préalable entre le polyisocyanate et l'HBPA est indispensable pour obtenir des propriétés de séchage améliorées.

Les exemples qui suivent illustrent des composés selon l'invention et leur utilisation dans des compositions du type durcisseur en phase aqueuse.

Dans ces exemples, on utilise comme matière première les produits RHODAFAC DV 6175 et RHODAFAC DV 6176 qui sont des mélanges de tensioactifs, vendus par la société RHODIA, constitués d'un mélange de mono et di

ester phosphate d'un mono éther d'alcool gras de polyéthylène glycol et d'acide phosphorique. L'alcool gras est une chaîne ramifiée constituée de 13 atomes de carbone en moyenne, et le nombre d'unités oxyde d'éthylène est centré sur 6 environ. Ces deux tensioactifs se distinguent par le rapport des composés mono, di ester et acide phosphorique.

Pour les compositions en phase aqueuse, la mesure de la taille des particules des polyisocyanates hydrophiles après dispersion dans l'eau se fait de la manière suivante : 5 g de formulation de polyisocyanate hydrophile sont ajoutés à 45 g d'eau distillée à température ambiante. Le milieu réactionnel est mis sous agitation 5 minutes à 400 tours / minutes à l'aide d'un module d'agitation de type hélices 4 pales.

La courbe de granulométrie est mesurée à l'aide d'un granulomètre à diffraction laser de type MALVERN mastersizer 2000.

EXEMPLE 31

Cet exemple concerne la préparation d'un composé X (phase 1); la préparation d'un composé selon l'invention à partir du composé X formé précédemment (phase 2); la préparation d'une composition ou formulation de type durcisseur selon l'invention en phase aqueuse (phase 3).

Phase 1

Le composé X préparé ici est un diol ester qui est le produit de la réaction d'un composé à fonction époxy avec un phosphate.

Dans un réacteur tricol équipé d'une agitation mécanique, d'ampoules d'addition, et à double enveloppe, on introduit 50,76 g de dibutyl phosphate (soit 0,242 mole / RN Cas 107 - 66 - 4). 29,94 g du 3,4 époxy cyclohexyle carboxylate de 3,4 époxy cyclohexane 1 méthyle (soit 0,119 mole / RN Cas 2386 - 87 - 0) sont ajoutés en 20 minutes à température ambiante sous agitation et sous atmosphère d'azote. La réaction est exothermique. La température du milieu réactionnel est amenée à 100°C. La cinétique de réaction est suivie par analyse Infra Rouge (I.R.) réalisée sur un prélèvement du milieu réactionnel.

La disparition des fonctions époxy est suivie à 788,76 $cm^{-1}$. L'apparition des fonctions hydroxyles est observée à 3392,96 $cm^{-1}$ et celle des fonctions esters phosphates à 1253,59 $cm^{-1}$. La fonction ester carboxylique du produit de départ est visualisée à 1732,88 $cm^{-1}$. On utilise comme référence la bande alkyle située à 2955 $cm^{-1}$. Le suivi cinétique est mesurée sur le ratio des fréquences 788,76 $cm^{-1}$ / 2955 $cm^{-1}$.

La réaction est terminée dès que l'analyse IR montre que la fonction époxy est ouverte à plus de 95% et que le diol ester attendu est bien obtenu.

L'ouverture de la fonction époxy pouvant se faire par attaque sur le carbone 3 ou le carbone 4, le produit final diol ester est un mélange de structures dont une est représentée ci-dessous.

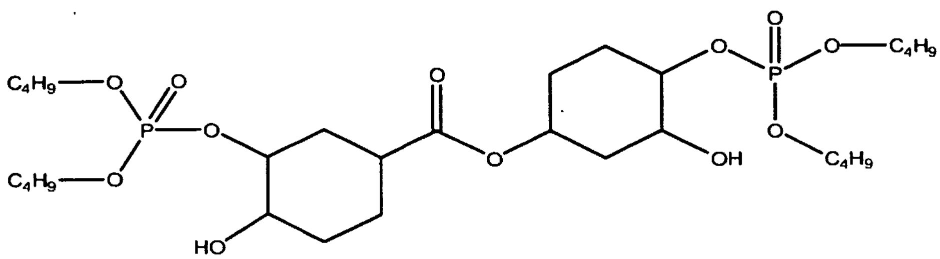

Phase 2

Dans un réacteur tricol à double enveloppe, équipé d'une agitation mécanique et d'ampoules d'addition on introduit 13,82g du diol ester obtenu à l'issue de la phase 1 (0,082 mole de fonctions hydroxyles) et 302 g de polyisocyanate isocyanurate TOLONATE HDT LV2 dont le titre NCO est de 0,543 mole de NCO pour 100 g et la viscosité à 25°C de 600 mPa.s.

Le ratio massique diol ester / polyisocyanate est de 4,6%.

La température du milieu réactionnel est alors montée à 100°C et le milieu réactionnel est laissé sous agitation pendant 5H30 à cette température. Après refroidissement le produit est collecté dans un flacon récepteur. La masse récupérée est de 315 g. La viscosité est de 1850 mPa.s à 25°C.

Le TOLONATE HDT LV2 étant une formulation de polyisocyanates comportant plusieurs structures et le produit issu de la phase 1 étant lui aussi un mélange de structures, le produit final obtenu est une formulation de polyisocyanates uréthanes esters dont une des structures est représentée ci après.

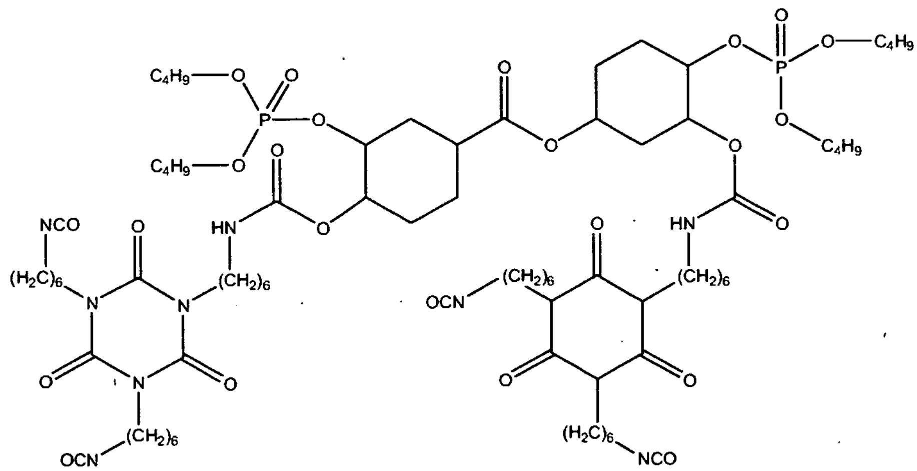

Phase 3

Dans un réacteur, on introduit successivement 90 g du produit issu de la phase 2, 1,92 g de N, N di méthyle cyclohexylamine, 4,04 g de tensioactif RHODOAFAC DV 6175 et 4,04g de tensioactif RHODAFAC DV 6176. Le mélange est agité à 40°C pendant 2 heures puis refroidi à température ambiante.

Le titre NCO de la formulation finale de type durcisseur à base de polyisocyanate hydrophile ainsi obtenue est de 0,466 mole de NCO pour 100 g, soit 19,57% poids.

La taille de particules mesurée après dispersion dans l'eau est centrée sur 0,1 micron.

EXEMPLE 32

Cet exemple concerne aussi la préparation d'un composé X (phase 1); la préparation d'un composé selon l'invention à partir du composé X formé précédemment (phase 2); la préparation d'une composition de type durcisseur selon l'invention en phase aqueuse (phase 3).

Phase 1

Le composé X préparé ici est un diol ester qui est le produit de la réaction d'un composé à fonction époxy avec un acide carboxylique.

Dans un réacteur tricol à double enveloppe équipé d'une agitation mécanique et d'ampoules d'addition, on introduit 20 g du 3,4 époxy cyclohexyle carboxylate de 3,4 époxy cylohexane 1 méthyle (soit 0,079mole / RN Cas 2386 - 87 - 0). 12,09 g d'acide propionique (0,165 mole) sont ajoutés en 20 minutes à température ambiante sous agitation et sous atmosphère d'azote. La réaction est exothermique. La température du milieu réactionnel est amenée à 100°C.

Après 3 heures à 100°C on observe par analyse Infra rouge que le composé époxy est ouvert à plus de 92% et que le diol attendu est bien obtenu. On laisse sous agitation à 100°C pendant encore 4 heures puis le milieu réactionnel est laissé refroidir à température ambiante puis mis en flacon.

Le produit est contrôlé par infra rouge :

- disparition des fonctions époxy (788,76 $cm^{-1}$).
- apparition des fonctions hydroxyles (3392,96 $cm^{-1}$)
- confirmation de la fonction ester carboxylique (1732,88 $cm^{-1}$).

L'ouverture de la fonction époxy pouvant se faire par attaque sur le carbone 3 ou le carbone 4, le produit final diol ester est un mélange de structures dont une est représentée ci-dessous.

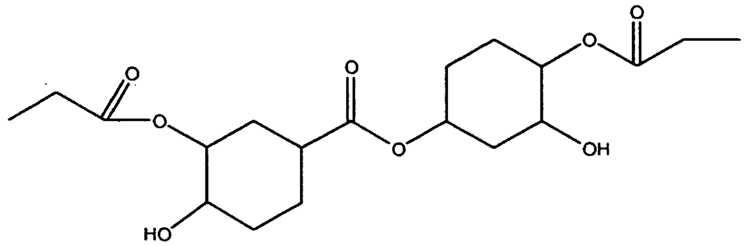

Phase 2

Dans un réacteur tricol à double enveloppe, équipé d'une agitation mécanique et d'ampoules d'addition on introduit 10,9 g (0,027 mole) du diol ester obtenu à l'issue de la phase 1 de masse moléculaire 400,47 et 303,8 g de polyisocyanate isocyanurate TOLONATE HDT LV2 de viscosité à 25°C de 600 mPa.s et dont le titre NCO est de 0,543 mole de NCO pour 100g.

Le ratio massique diol ester / polyisocyanate est de 4 %. On ajoute 15 microlitres de dibutyl dilaurate d'étain. La température du milieu réactionnel est alors portée à 110 °C et le milieu réactionnel est laissé sous agitation pendant 8H à cette température. Le titre NCO passe de 0,525 mole pour 100 g à 0,492 moles de NCO pour 100 g après 8 H de réaction. La consommation des fonctions NCO représente 6,3 % en fin de réaction.

Après refroidissement, le produit est collecté dans un flacon. La masse récupérée est de 314 g.

Le titre NCO de la formulation finale de polyisocyanates est de 0,492 moles de NCO pour 100 g. La viscosité est de 5187 mPa.s à 25°C.

Le TOLONATE HDT LV2 étant une formulation de polyisocyanates comportant plusieurs structures et le produit issu de la phase 1 étant lui aussi un mélange de structures, le produit final obtenu est une formulation de polyisocyanates uréthanes esters dont une des structures est représentée ci après.

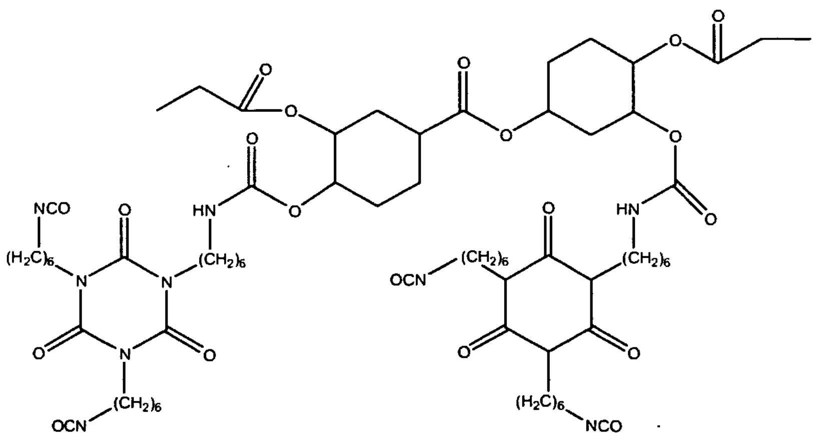

Phase 3

Dans un réacteur, on introduit successivement 90 g du produit issu de la phase 2, 1,92 g de N, N di méthyle cyclohexylamine, 4,04 g de tensioactif RHODAFAC DV 6175 et 4,04g de tensioactif RHODAFAC DV 6176. Le mélange est agité à 40°C pendant 2 heures puis refroidi à température ambiante.

Le titre NCO de la formulation finale de type durcisseur à base de polyisocyanate hydrophile est de 0,418 mole de NCO pour 100 g, soit 17,56 % en poids. La taille de particules mesurée après dispersion dans l'eau est centrée sur 0,1 micron.

EXEMPLE COMPARATIF 33

Cet exemple décrit la préparation d'une formulation à partir d'un TOLONATTE HDT LV2 non modifié, à titre comparatif avec les formulations obtenues à l'issue des phases 3 des exemples 31 et 32.

On introduit dans un réacteur 90 g de TOLONATE HDT LV2, 1,92 g de N, N di méthyle cyclohexylamine, 4,04 g de tensioactif RHODAFAC DV 6175 et 4,04 g de tensioactif RHODAFAC DV 6176 et 30 g d'acétate de méthoxypropyle. Le mélange est agité à 40°C pendant 2 heures puis refroidi à température ambiante.

Le titre NCO de la formulation finale obtenue est de 0,36 mole pour 100g.

EXEMPLE 34

Cet exemple illustre l'utilisation de compositions durcisseurs comparatives et selon l'invention dans des applications de type vernis.

On réalise une formulation de vernis en utilisant comme matières premières une dispersion d'un poyol acrylique dont le taux de fonctions hydroxyles est de 4,8 % et les formulations obtenues à l'issue des phases 3 des exemples 31 et 32 et celle de l'exemple comparatif 33. Le ratio molaire NCO / OH utilisé dans est de 1,2.

Dans un réacteur on ajoute successivement 50 g de polyol, 14,54 g de formulation de l'exemple 21 obtenue à l'issue de la phase 3, 3,91 g d'eau et 0,012g de di butyl di laurate d'étain (DBTL). Le mélange est agité 2 minutes sous agitation moyenne. La formulation obtenue est homogène et aucun agrégat n'est visible. Il n'y a pas formation de mousses. L'émulsion est laissée au repos durant 15 minutes avant toute application sur le support.

On procède de la même manière pour la formulation de durcisseur polyisocyanate hydrophile de l'exemple 32 obtenues à l'issue de la phase 3. Dans un réacteur on ajoute successivement 50 g de polyol, 16,21 g de ladite formulation, 4,01 g d'eau et 0,011 g de di butyl di laurate d'étain (DBTL). Le mélange est agité 2 minutes sous agitation moyenne. La formulation obtenue est homogène et aucun agrégat n'est visible. Il n'y a pas formation de mousses. L'émulsion est laissée au repos durant 15 minutes avant toute application sur le support.

La formulation comparative est préparée de la même manière en ajoutant dans un réacteur 50 g de polyol, 18,77 g de formulation de l'exemple comparatif 23, 4,17 g d'eau, et 0,014 g de DBTL. L'émulsion est laissée au repos durant 15 minutes avant toute application sur le support.

Les formulations sont ensuite appliquées sur plaques de verre. Pour étaler le film, une raclette de 150 $\mu$m d'épaisseur et un tire-film sont utilisés. La raclette est posée sur la plaque, est remplie par l'émulsion et est poussée par le tire-film à une vitesse de 24 mm/s.

Après 15 minutes d'évaporation à température ambiante, les plaques sont placées dans une étuve à 60°C pendant 30 minutes. La cuisson terminée, les propriétés des films sont alors mesurées après 1, 3 et 24 heures de séchage à température ambiante.

Les résultats sont présentés dans le tableau 21 ci après.

Tableau 21

| Durcisseur | Epaisseur de film en microns | Dureté 1H | Dureté 3H | Dureté 24H | Brillance |
|---|---|---|---|---|---|
| Ex 31 | 33 | 68 | 108 | 315 | 92 |
| Ex 32 | 32 | 82 | 130 | 315 | 90 |
| Ex 33 comparatif | 29 | 27 | 54 | 305 | 92 |

Les formulations de durcisseurs polyisocyanates hydrophiles de l'invention obtenues à partir du TOLONATE HDT LV2 modifié par réaction avec des groupes cycloaliphatiques présentent de meilleures propriétés de films que le même polyisocyanate non modifié. On remarque tout particulièrement que la dureté initiale (dureté à 1 et 3 heures) est beaucoup plus élevée.

## Revendications

**1.** Composé à fonctionnalité isocyanate, présentant une fonctionnalité moyenne supérieure à 2, résultant de la réaction d'un (poly)isocyanate de fonctionnalité moyenne supérieure à 2 avec au moins un composé X comprenant :

- soit au moins une fonction $B(H)_n$ dans laquelle :

n est un nombre égal à 1 ou 2,
H est un hydrogène labile et
B désigne O, S, N, N étant un azote primaire ou secondaire, -C(=O)-O, -C(=O)-N, ou encore les groupes

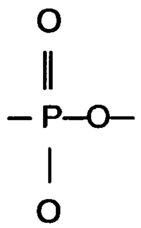
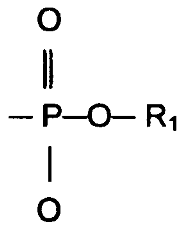
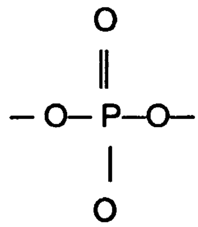

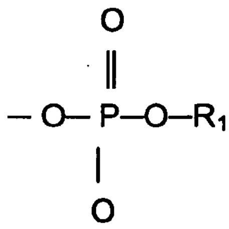
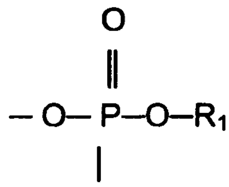

$R_1$ désignant un radical alkyle ou aralkyle, éventuellement ramifiée, ou une chaîne alkyle interrompue par un hétéroatome;
- soit au moins une fonction $B'(H)_{n'}$ dans laquelle :

n' est un nombre égal à 1, 2 ou 3,
H est un hydrogène labile et B' désigne $-SiR_2R_3R_4$, $R_2$, $R_3$, $R_4$ représentant l'oxygène, un radical alkyle portant une fonction réactive avec le (poly)isocyanate, un radical aralkyle, aryle, -O-alkyle ou -O-aralkyle, le nombre de radicaux $R_2$, $R_3$, $R_4$ étant tel que n' puisse bien vérifier la condition ci-dessus;
X étant en outre un composé cycloaliphatique, aromatique ou hétérocyclique comprenant au moins deux cycles; au moins une fonction B(H)n ou B'(H)n', de préférence toutes, sont liées directement à un carbone du cycle du composé X et avec comme condition
que la réaction précitée soit conduite avec un rapport pondéral composé X/[composé X + (poly)isocyanate] d'au plus 50%.

2. Composé selon la revendication 1, **caractérisé en ce qu'**il présente une fonctionnalité d'au moins 2,5 , plus particulièrement d'au moins 3,5.

3. Composé selon la revendication 1 ou 2, **caractérisé en ce qu'**il résulte d'une réaction avec un composé X à structure rigide.

4. Composé selon l'une des revendications précédentes, **caractérisé en ce qu'**il résulte d'une réaction avec un composé X dans lequel la fonction $B(H)_n$ est la fonction OH.

5. Composé selon la revendication 4, **caractérisé en ce qu'**il résulte d'une réaction avec un composé X qui est choisi parmi :

- les bisphénols, notamment A et F, leurs dérivés hydrogénés, leurs dérivés polyphénoliques à pont éther et les dérivés hydrogénés de ceux-ci;
- les dérivés du dicyclopentadiène et du tricyclopentadiène;
- les dérivés de la série des terpènes;
- les cyclanes à fonction OH.

6. Composé selon la revendication 4, **caractérisé en ce qu'**il résulte d'une réaction avec un composé X qui est le produit de la réaction d'un acide carboxylique avec un composé à fonction hydroxyle masquée.

7. Composé selon la revendication 4, **caractérisé en ce qu'**il résulte d'une réaction avec un composé X qui est le produit de la réaction d'un acide carboxylique avec un composé à fonction époxy.

8. Composé selon la revendication 4 **caractérisé en ce que** le composé X est le produit de la réaction d'un composé comprenant au moins une fonction époxy avec un phosphate de formule $(O)P(OR_6)(OR_7)(OR_8)$ dans laquelle $R_6$,

$R_7$, $R_8$, sont identiques ou différents et désignent l'hydrogène, un radical alkyle, linéaire ou ramifié, un radical cycloaliphatique, un radical aromatique, un radical aralkyle, une chaîne polyoxyalkylène dont le nombre de motifs oxyalkylènes linéaires ou ramifiés est compris entre 1 et 25 et dont le nombre de carbones de la chaîne alkylène est compris entre 2 et 6, cette chaîne polyoxyalkylène pouvant être, de préférence, substituée sur ses fonctions terminales par une chaîne alkyle, linéaire ou ramifiée, ou par une chaîne aralkyle, éventuellement ramifiée; et avec comme condition qu'au moins un de $R_6$, $R_7$, $R_8$ soit un atome d'hydrogène.

**9.** Composé selon la revendication 4 **caractérisé en ce que** le composé X est le produit de la réaction d'un composé à fonction époxy avec un polyaminoéther de formule (3) $R_9$-[-O-$CH_2$-$CH_2$-O-]$_p$-$NH_2$ ou

(3') 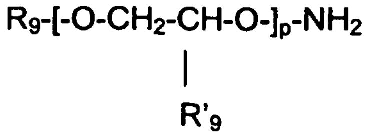

ou encore de formule (4) $R_9$-[-O-$CH_2$-$CH_2$-O-]$_q$-CH(CH)-$CH_2$-$NH_2$, $R_9$ étant l'hydrogène, un radical alkyle, un radical $CH_2CH_2NH_2$ ou $CH_2CH(CH_3)NH_2$ , $R'_9$ étant un radical alkyle, p et q étant des nombres entiers compris entre 2 et 10.

**10.** Composé selon l'une des revendications précédentes, **caractérisé en ce qu'**il résulte d'une réaction d'un composé X avec un (poly)isocyanate qui présente au moins un cycle isocyanurate ou un motif biuret ou une fonction allophanate ou encore une fonction acylurée et qui peut être obtenu par d'homo ou d'hétérocondensation de monomères choisis parmi les monomères isocyanates aliphatiques, cycloaliphatiques, arylaliphatiques, aromatiques et hétérocycliques.

**11.** Procédé de préparation d'un composé selon l'une des revendications précédentes, **caractérisé en ce qu'**on fait réagir le (poly)isocyanate précité avec le composé X précité dans un rapport pondéral composé X/[composé X + (poly)isocyanate] d'au plus 40%, plus particulièrement d'au plus 25%.

**12.** Procédé selon la revendication 10, **caractérisé en ce qu'**on fait réagir le (poly)isocyanate précité avec le composé X précité en quantité telle que le ratio molaire $B(H)_n$/NCO soit compris entre 1 et 50 %.

**13.** Composition du type durcisseur, **caractérisée en ce qu'**elle comprend au moins un composé selon l'une des revendications 1 à 10.

**14.** Composition selon la revendication 13, **caractérisée en ce qu'**elle comprend un additif hydrophile du type non réactif.

**15.** Composition selon la revendication 13, **caractérisée en ce qu'**elle comprend un additif hydrophile greffé sur le composé à fonctionnalité isocyanate.

**16.** Composition selon la revendication 13, **caractérisée en ce qu'**elle comprend en outre un polyisocyanate

**17.** Procédé de préparation d'un revêtement sur un substrat, **caractérisé en ce qu'**on fait réagir une composition selon l'une des revendications 13 à 16 avec un composé porteur d'au moins une fonction à hydrogène mobile choisie parmi les fonctions hydroxyle, amine primaire, amine secondaire et la fonction SH ou avec un composé contenant des fonctions précurseurs susceptibles de libérer des fonctions hydroxyles; puis on applique le mélange obtenu sur le substrat.

**18.** Procédé selon la revendication 17, **caractérisé en ce que** le composé porteur d'au moins une fonction à hydrogène mobile est un polyol choisi parmi les polymères acryliques, polyester ou polyuréthanes ou des hybrides de ces polymères.

**19.** Utilisation d'un composé selon l'une des revendications 1 à 10 comme accélérateur de séchage dans un procédé de préparation d'un revêtement sur un substrat dans lequel on mélange une composition comprenant ledit composé avec un composé porteur d'au moins une fonction à hydrogène mobile choisie parmi les fonctions hydroxyle, amine primaire, amine secondaire et la fonction SH et où l'on dépose le mélange ainsi obtenu sur le substrat.

**Patentansprüche**

1. Verbindung mit Isocyanat-Funktionalität, die eine mittlere Funktionalität größer als 2 aufweist, die aus der Reaktion eines (Poly)isocyanats einer mittleren Funktionalität größer als 2 mit mindestens einer Verbindung X resultiert, die umfasst:

   - entweder mindestens eine Funktion $B(H)_n$, bei der:

     n eine Zahl gleich 1 oder 2 ist,
     H ein labiler Wasserstoff ist, und
     B O, S, N bezeichnet, wobei N ein Primär- oder Sekundärsttckstoff ist, -C(=O)-O, -C(=O)-N oder auch die Gruppen

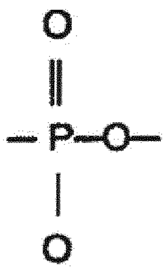
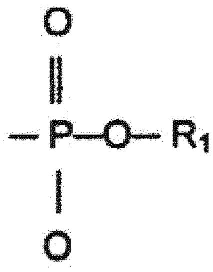
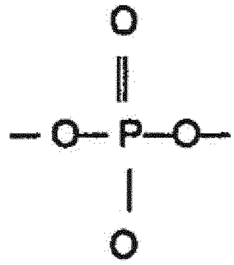
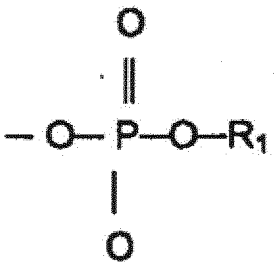
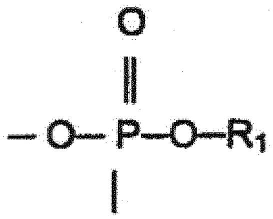

   wobei $R_1$ ein Alkyl- oder Aralkylradikal, gegebenenfalls verzweigt, oder eine durch ein Heteroatom unterbrochene Alkylkette bezeich-net,
   - oder mindestens eine Funktion $B'(H)_n$, bei der:

     n' eine Zahl gleich 1, 2 oder 3 ist,
     H ein labiler Wasserstoff ist und $B'$-$SiR_2R_3R_4$ bezeichnet, wobei $R_2$, $R_3$, $R_4$ Sauersoff bezeichnen, ein Alkylradikal eine reaktive Funktion mit dem (Poly)isocyanat, einen Aralkylradikal, Aryl, -O-alkyl oder -O-aralkyl trägt, wobei die Anzahl der Radikale $R_2$, $R_3$, $R_4$ derart ist, dass n' die oben angegebene Bedingung verifizieren kann;
     X außerdem eine cycloaliphatische, aromatische oder heterocyclische Verbindung ist, die mindestens zwei Ringe umfasst; wobei mindes-tens eine Funktion B(H)n oder B'(H)n', vorzugsweise alle, direkt an ein Kohlenstoff des Ringes der Verbindung X gebunden sind und mit der Bedingung,
     dass die vorerwähnte Reaktion mit einem Gewichtsverhältnis Verbindung X/[Verbindung X + (Poly)isocyanat] von höchstens 50% durch geführt wird.

2. Verbindung nach Anspruch 1, **dadurch gekennzeichnet, dass** sie eine Funktionalität von mindestens 2,5, insbesondere von mindestens 3,5 aufweist.

3. Verbindung nach Anspruch 1 oder 2, dadurch gekenntzeichnet, dass sie aus einer Reaktion mit einer Verbindung X mit starrer Struktur resultiert.

4. Verbindung nach einem der vorhergehenden Ansprüche, **dadurch gekennzeichnet, dass** sie aus einer Reaktion mit einer Verbindung X resultiert, in der die Funktion $B(H)_n$ die Funktion OH ist.

**5.** Verbindung nach Anspruch 4, **dadurch gekennzeichnet, dass** sie aus einer Reaktion mit einer Verbindung X resultiert, die ausgewählt ist aus:

- den Bisphenolen, insbesondere A und F, ihren hydrierten Derivaten, ihren Polyphenolderivaten mit Etherbrücke und den hydrierten Derivaten derselben;
- den Derivaten des Dicydopentadiens und des Tricydopentadiens;
- den Derivaten der Reihe der Terpene;
- den Cyclanen mit OH-Funktion.

**6.** Verbindung nach Anspruch 4, dadurch gekenntzeichnet, dass sie aus einer Reaktion mit einer Verbindung X resultiert, die das Produkt der Reaktion einer Carbonsäure mit einer Verbindung mit markierter Hydroxyfunktion ist.

**7.** Verbindung nach Anspruch 4, **dadurch gekennzeichnet, dass** sie aus einer Reaktion mit einer Verbindung X resultiert, die das Produkt der Reaktion einer Carboxylsäure mit einer Verbindung mit Epoxyfunktion ist.

**8.** Verbindung nach Anspruch 4, **dadurch gekennzeichnet, dass** die Verbindung X das Produkt der Reaktion einer Verbindung, die mindestens eine Epoxyfunktion umfasst, mit einem Phosphat der Formel $(O)P(OR_6)(OR_7)(OR_8)$, in der $R_6$, $R_7$, $R_8$ identisch oder unterschiedlich sind und Wasserstoff, ein Alkylradikal, linear oder verzweigt, ein cycloaliphatisches Radikal, ein aromatisches Radikal, ein Aralkylradikal, eine Polyoxyalkylenkette bezeichnen, deren Anzahl von Oxyalkyleneinheiten, linear oder verzweigt, zwischen 1 und 25 liegt und dessen Kohlenstoffzahl der Alkylenkette zwischen 2 und 6 liegt, wobei diese Polyoxyalkylenkette vorzugsweise an seinen Endfunktionen durch eine Alkylkette, linear oder verzweigt oder durch eine Aralkylkette, gegebenenfalls verzweigt, substituiert werden kann; und mit der Bedingung, dass mindestens eines von $R_6$, $R_7$, $R_8$ ein Wasserstoffatom ist.

**9.** Verbindung nach Anspruch 4, **dadurch gekennzeichnet, dass** die Verbindung X das Produkt aus der Reaktion einer Verbindung mit Epoxyfunktion mit einem Polyaminether der Formel (3) $R_9$-[-O-$CH_2$-$CH_2$-O-]$_p$-$NH_2$ oder

(3')
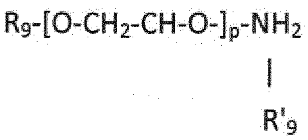

oder auch der Formel (4) $R_9$-[-O-$CH_2$-$CH_7$-O-]$_q$-CH(CH)-$CH_2$-$NH_2$ ist, wobei $R_9$ Wasserstoff, ein Alkylradikal, ein Radikal $CH_2CH_2NH_2$ oder $CH_2CH(CH_3)NH_2$ ist, $R'_9$ ein Alkylradikal ist, p und q ganze Zahlen zwischen 2 und 10 sind.

**10.** Verbindung nach einem der vorhergehenden Ansprüche, **dadurch gekennzeichnet, dass** sie aus einer Reaktion einer Verbindung X mit einem (Poly)isocyanat resultiert, das mindestens einen Isocyanuratring oder eine Biuret-Einheit oder eine Allophanatfunktion oder auch eine Acylharnstofffunktion aufweist und dass durch Homo- oder Heterokondensation von Monomeren erhalten werden kann, die ausgewählt sind aus den aliphatischen, cycloaliphatischen, arylaliphatischen, aromatischen und heterocyklischen Isocyanatmonomeren.

**11.** Verfahren zur Herstellung einer Verbindung nach einem der vorhergehenden Ansprüche, **dadurch gekennzeichnet, dass** man das vorerwähnte (Poly)isocyanat mit der vorerwähnten Verbindung X mit einem Gewichtsverhältnis Verbindung X/[Verbindung X + (Poly)isocyanat] von höchstens 40%, insbesondere höchstens 25% reagieren lässt.

**12.** Verfahren nach Anspruch 10, **dadurch gekennzeichnet, dass** man das vorerwähnte (Poly)isocyanat mit der vorerwähnten Verbindung X in einer Menge reagieren lässt, derart, dass das Molverhältnis $B(H)_n$/NCO zwischen 1 und 50% liegt.

**13.** Zusammensetzung des Typs Härter, **dadurch gekennzeichnet, dass** sie mindestens einer Verbindung nach einem der Ansprüche 1 bis 10 umfasst.

**14.** Zusammensetzung nach Anspruch 13, **dadurch gekennzeichnet, dass** sie ein nichtreaktives hydrophiles Additiv umfasst.

**15.** Zusammensetzung nach Anspruch 13, **dadurch gekennzeichnet, dass** sie ein hydrophiles Additiv umfasst, das auf die Verbindung mit Isocyanatfunktionalität aufgepfropft ist.

**16.** Zusammensetzung nach Anspruch 13, **dadurch gekennzeichnet, dass** sie außerdem ein Polyisocyanat umfasst.

**17.** Verfahren zur Herstellung einer Beschichtung auf einem Substrat, **dadurch gekennzeichnet, dass** man eine Zusammensetzung nach einem der Ansprüche 13 bis 16 mit einer Trägerverbindung mit mindestens einer mobilen Wasserstofffunktion, die ausgewählt ist aus den Hydroxyfunktionen, den primären Aminfunktionen, den sekundären Aminfunktionen und der Funktion SH, oder mit einer Verbindung reagieren lässt, die Precursorfunktionen enthält, die fähig sind, Hydroxyfunktionen freizusetzen; dann die erhaltene Mischung auf das Substrat aufgebracht wird.

**18.** Verfahren nach Anspruch 17, **dadurch gekennzeichnet, dass** die Trägerfunktion der mindestens einen mobilen Wasserstofffunktion ein Polyol ist, das ausgewählt ist aus den Acrylpolymeren, aus Polyestern oder aus Polyurethanen oder Hybriden dieser Polymere.

**19.** Verwendung einer Verbindung nach einem der Ansprüche 1 bis 10 als Trocknungsbeschleuniger in einem Verfahren zur Herstellung einer Beschichtung auf einem Substrat, bei der eine die Verbindung enthaltende Zusammensetzung mit einer Trägerverbindung mindestens einer mobilen Wasserstofffunktion gemischt wird, die aus den Hydroxyfunktionen, den primären Aminfunktionen, den sekundären Aminfunktionen und der Funktion SH ausgewählt ist, und bei der die so erhaltene Mischung auf dem Substrat abgeschieden wird.

## Claims

**1.** Compound having isocyanate functionality, having an average functionality of greater than 2, resulting from the reaction of a (poly)isocyanate of average functionality of greater than 2 with at least one compound X, comprising:

- either at least one $B(H)_n$ function in which:

  n is a number equal to 1 or 2,
  H is an unstable hydrogen and
  B designates O, S, N, N being a primary or secondary nitrogen, -C(=O)-O, -C(=O)-N, or also the groups

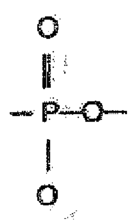
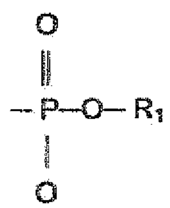
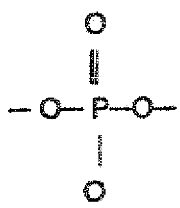
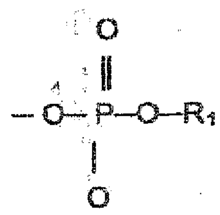
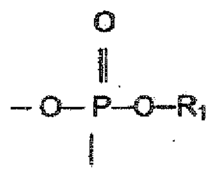

  $R_1$ designating an alkyl or aralkyl radical, possibly branched, or an alkyl chain interrupted by a heteroatom;

- or at least one $B'(H)_{n'}$ function in which:

  n' is a number equal to 1, 2 or 3,
  H is an unstable hydrogen and B' designates $-SiR_2R_3R_4$, $R_2$, $R_3$, $R_4$ representing oxygen, an alkyl radical which carries a reactive function with the (poly)isocyanate, an aralkyl, aryl, -O-alkyl or- O-aralkyl radical, the number of radicals $R_2$, $R_3$, $R_4$ being such that the above condition is not readily able to be verified;
  X being in addition a cycloaliphatic, aromatic or heterocyclic compound, comprising at least two cycles; at least one B(H)n or B'(H)n' function, preferably all of them, are bonded directly to a carbon of the cycle of compound X and with as a condition that the aforementioned reaction is conducted with a weight ratio of

compound X/ [compound X + (poly)isocyanate] of at most 50%.

2. Compound according to claim 1, **characterised in that** it has a functionality of at least 2.5, more particularly of at least 3.5.

3. Compound according to claim 1 or 2, **characterised in that** it results from a reaction with a compound X having a rigid structure.

4. Compound according to one of the preceding claims, **characterised in that** it results from a reaction with a compound X in which the $B(H)_n$ function is the OH function.

5. Compound according to claim 4, **characterised in that** it results from a reaction with a compound X which is chosen from:

- the bisphenols, in particular A and F, their hydrogenated derivatives, their polyphenolic derivatives having an ether bridge and the hydrogenated derivatives of the latter;
- the derivatives of dicyclopentadiene and of tricyclopentadiene;
- the derivatives of the series of terpenes;
- the cyclanes having an OH function.

6. Compound according to claim 4, **characterised in that** it results from a reaction with a compound X which is the product of the reaction of a carboxylic acid with a compound having a masked hydroxyle function.

7. Compound according to claim 4, **characterised in that** it results from a reaction with a compound X which is the product of the reaction of a carboxylic acid with a compound having an epoxy function.

8. Compound according to claim 4, **characterised in that** the compound X is the product of the reaction of a compound comprising at least one epoxy function with a phosphate of formula $(O)P(OR_6)(OR_7)(OR_8)$ in which $R_6$, $R_7$, $R_8$ are identical or different and designate hydrogen, an alkyl radical, linear or branched, a cycloaliphatic radical, an aromatic radical, an aralkyl radical, a polyoxyalkylene chain, the number of linear or branched oxyalkylene units of which is between 1 and 25 and the number of carbons of the alkylene chain of which is between 2 and 6, this polyoxyalkylene chain being able to be, preferably, substituted on the terminal functions thereof by an alkyl chain, linear or branched, or by an aralkyl chain, possibly branched; and with as a condition that at least one of $R_6$, $R_7$, $R_8$ is a hydrogen atom.

9. Compound according to claim 4, **characterised in that** the compound X is the product of the reaction of a compound having an epoxy function with a polyaminoether of formula (3) $R_9\text{-[-O-}CH_2\text{-}CH_2\text{-O-]}_p\text{-}NH_2$
or

(3') 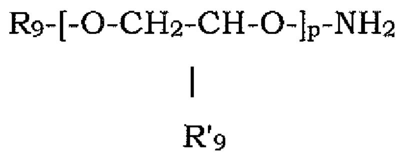

or also of formula (4) $R_9\text{-[-O-}CH_2\text{-}CH_2\text{-O-]}_q\text{-CH(CH)-}CH_2\text{-}NH_2$, $R_9$ being hydrogen, an alkyl radical, a $CH_2CH_2NH_2$ or $CH_2CH(CH_3)NH_2$ radical, R'g being an alkyl radical, p and q being whole numbers between 2 and 10.

10. Compound according to one of the preceding claims, **characterised in that** it results from a reaction of a compound X with a (poly)isocyanate which has at least one isocyanurate cycle or a biuret unit or an allophanate function or also an acyl urea function and which can be obtained by homo- or heterocondensation of monomers chosen from the aliphatic, cycloaliphatic, arylaliphatic, aromatic and heterocyclic isocyanate monomers.

11. Method for preparing a compound according to one of the preceding claims, **characterised in that** the aforementioned (poly)isocyanate is made to react with the aforementioned compound X in a weight ratio of compound X/ [compound X + (poly)isocyanate] of at most 40%, more particularly of at most 25%.

12. Method according to claim 10, **characterised in that** the aforementioned (poly)isocyanate is made to react with the

aforementioned compound X in a quantity such that the molar ratio $B(H)_n/NCO$ is between 1 and 50%.

**13.** Composition of the hardening agent type, **characterised in that** it comprises at least one compound according to one of the claims 1 to 10.

**14.** Composition according to claim 13, **characterised in that** it comprises a hydrophilic additive of the non-reactive type.

**15.** Composition according to claim 13, **characterised in that** it comprises a hydrophilic additive grafted onto the compound having isocyanate functionality.

**16.** Composition according to claim 13, **characterised in that** it comprises in addition a polyisocyanate.

**17.** Method for preparing a coating on a substrate, **characterised in that** a composition according to one of the claims 13 to 16 is made to react with a compound which carries at least one mobile hydrogen function, chosen from the hydroxyl, primary amine, secondary amine functions and the SH function or with a compound containing precursor functions which are able to release hydroxyl functions; then the obtained mixture is applied on the substrate.

**18.** Method according to claim 17, **characterised in that** the compound which carries at least one mobile hydrogen function is a polyol chosen from acrylic, polyester or polyurethane polymers or hybrids of these polymers.

**19.** Use of a compound according to one of the claims 1 to 10 as drying accelerator in a method for preparing a coating on a substrate in which a composition comprising said compound is mixed with a compound which carries at least one mobile hydrogen function, chosen from hydroxyl, primary amine, secondary amine functions and the SH function and where the thus obtained mixture is deposited on the substrate.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6730405 B
- WO 9731960 A
- FR 2855768 A1
- US 4663377 A


### Littérature non-brevet citée dans la description


- **HOUBEN WEYL.** Methoden der Organischen Chemie. Georg Thieme Verlag, 1983